# EUROPEAN PATENT APPLICATION

(11) **EP 4 397 669 A1**
(43) Date of publication of application: **10.07.2024**
(21) Application number: 23150592.6
(22) Date of filing: 06.01.2023
(51) Int. Cl.: C07H 21/02, A61K 31/7084

(54) **PHOTOCAGED CAP ANALOGS FOR THE 5'-END OF RNAS**

(71) Applicant: Westfälische Wilhelms-Universität Münster, 48149 Münster (DE)
(72) Inventor: RENTMEISTER, Andrea, 48161 Münster (DE); BOLLU, Amarnath, 48145 Münster (DE); SCHEPERS, Helena, 48155 Münster (DE); KLÖCKER, Nils, 48149 Münster (DE); SPACEK, Petr, 48268 Greven (DE); WEISSENBÖCK, Florian, 48161 Münster (DE)
(74) Representative: Michalski Hüttermann & Partner Patentanwälte mbB

(57) **Abstract**

The present invention relates to a 5'-cap analog comprising a photocleavable group at a positively charged nitrogen of the N7-position of guanosine or the N1-position of adenosine. The present invention further relates to a photocleavable nucleoside or nucleotide analog or salts thereof, comprising a photocleavable group at the N7-position of guanosine, at the N1-position of adenosine, or at the N3-position of cytidine.

## Description

The present invention relates to the field of photocleavable cap analogs for the 5'-end of eukaryotic messenger RNAs and bacterial RNAs.

Almost all eukaryotic messenger RNAs (mRNAs) are modified at their 5'-ends by a cap that is synthesized via addition of a 7-methylguanosine attached via a 5'-5' triphosphate bridge to the first transcribed nucleotide of the mRNA chain. In some species, including mammals, additional methylations occur, mainly at the 2'-O position of the first or the first and second nucleotides of the transcript, denoted cap 1 and cap 2, respectively, where the not methylated cap then is denoted cap 0. The cap structure plays a pivotal role in mRNA metabolism, and only those mRNAs that carry a cap structure are active in translation, i.e. serve as template for protein synthesis. Since the 5'-cap plays a fundamental role in RNA metabolism, modifying the 5'-cap has emerged as a tool for modulation of protein expression and investigation of biological processes.

Also bacterial RNA is modified at the 5'-end by a cap. The predominant dinucleoside tetraphosphates (Np₄Ns) in bacteria are Ap4A, Gp4A, Cp4A, and Up4A. These dinucleoside tetraphosphates are particularly produced in bacterial cells experiencing the stress. These are considered as a signal of stress; therefore, they are known as alarmones. These NpₙNs can be incorporated at the 5'-cap of RNAs using two types of RNA polymerases (RNAPs; bacteriophage T7 and *E. coli*) in *in vitro* transcription.

Cap analogs with different modifications have been developed. A widely used modified cap is the anti-reverse cap analog (ARCA), a modified cap analog in which the 3' OH group is replaced with OCH₃. Because of this substitution, the RNA polymerase can only initiate transcription with the remaining hydroxyl group thus forcing ARCA incorporation in the forward orientation. As a result, a stimulatory effect on translation is achieved. The so-called second-generation ARCAs include various modifications of the 5'-5'-phosphate bridge.

WO 2017/053297 A1 discloses 5'-capped RNAs, and methods and compositions for synthesizing 5'-capped RNAs. The described cap containing initiating oligonucleotide primers have natural or modified Cap 0, Cap 1, Cap 2 or trimethylguanosine-Cap (TMG-Cap) structures.

Strategies to allow control of location and duration of translation via caged nucleic acids have been developed. For this, numerous positions on nucleobases have been targeted, involving formal substitution of a hydrogen atom with a photocaging group. A photocontrollable cap (PC cap) usable to control the translation of mRNA in a reversible manner through illumination with light was described (Ogasawara, ACS Chem Biol. 2017, 12, 351-356). While introduction of a phenylazo group into the C8-position of guanosine (Ogasawara et al., Chem. Lett. 39, 956-957) resulted in a thermally unstable isomer, modification of the C2 amine resulted in a photoisomerisable cap. Via *cis-trans* photoisomerization, the 2-meta-methyl-phenylazo cap (mMe-2PA-cap) in the *trans* form silenced translation whereas the *cis* form provided translated protein. Such photocontrollable caps, however, are an artificial compound in mRNA metabolism and will not yield the native nucleoside.

In eukaryotic cells, during the capping steps of mRNA, guanine MTase with its co-substrate, *S*-adenosyl-*L*-methionine (SAM), methylates the N7-position of the 5'-cap (G(5')ppp(5')RNA) and generate a positive charge on N7G (m⁷G(5')ppp(5')RNA). The positive charge on the N7-position of the guanosine (N7G) of the cap interacts with π-electron clouds of several cap binding proteins and acts as a recognition site. For example, eukaryotic translation initiation factor (eIF4E) has a 1000-fold increased affinity to the methylated cap compared to its unmethylated precursor.

The N7-position of guanosine and the N1 position of adenosine were used for chemical modification with a photolabile caging group (Anhäuser et al., Angew. Chem. Int. Ed., 2020, 59, 3161-3165). While aryl ketones such as benzophenone and α-hydroxyalkyl ketone yielded photolabile caging groups if installed at the N7-position of guanosine or the N1 position of adenosine, common photocaging groups derived from the *ortho*-nitrobenzyl moiety were not suitable and did not yield the desired photocleavage to release guanosine. Further, the irradiation needed to remove benzophenone is not biocompatible. Thus, a control of the translation is not yet possible via the N7-position of the guanosine.

Alternative strategies used a photocleavable group fused via a carbamate group to the N2 position of the guanosine of the 5'-cap structure (Klöcker et al., Nature Chemistry, Vol. 14, 2022, 905-913; Bollu et al., Angew. Chem. Int. Ed. 2023, e202209975 (doi.org/10.1002/anie.202209975)). The carbamate linkage to the photocleavable group allows to restore the native cap by irradiation and generates bio-compatible carbon dioxide as by-product. However, the N7-position of guanosine has a larger impact on translation compared to the N2-position.

Therefore, there remains a need to provide a photocleavable 5'-cap analog providing a photocleavable group at the N7-position of guanosine.

This object is achieved by a photocleavable 5'-cap analog according to formulas (I) or (II) as given as follows or salts thereof, wherein the 5'-cap analog comprises a photocleavable group PC-Cou at the N7-position of guanosine or the N1-position of adenosine: 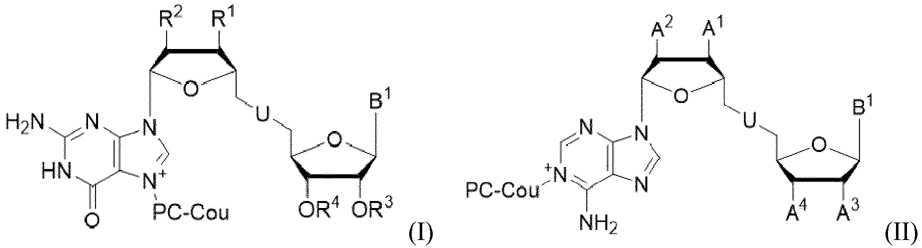 wherein:
- R¹, R²: are independently H, OH or OCH₃;
- R³: is H or CH₃;
- R⁴: is H or an element according to formula (III) 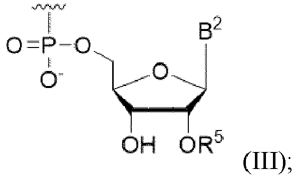
wherein R⁵ is H or CH₃;
- U: is a modified or unmodified oligophosphate or methyleneoligophosphate group;
- A¹: is R¹ or OR⁴;
- A²: is R² or OR³;
- A³: is OR³ or R²;
- A⁴: is OR⁴ or R¹;
- B¹, B²: is a modified or unmodified nucleobase; and
- PC-Cou: is a photocleavable coumarin group.

It was surprisingly found that a coumarin group can be cleaved completely from the positively charged nitrogen in nucleobases, exemplified with the N7-position of guanosine (N7G) and the N1-position of adenosine (N1A) under biologically compatible conditions. This provides that a photo-uncaging of a cap modified at the biologically relevant endocyclic N1 and N7 nitrogens in purines is possible under biological conditions. The N1- or N7-modified 5'-cap analogs enable highly efficient blocking and fast, residue-free photo-uncaging of the 5'-cap.

Cleavage of the coumarin group from the N7-position of guanosine initially results in an unmethylated cap that is, other than the naturally occurring N7-methylated cap, not translated. However, in mammalian cells (and in model organisms such as frog, moth, and zebrafish), the native cap is quickly restored through cellular N7-methylation and thereupon translation occurs. This enables a temporal and spatial control of protein biosynthesis. The N7-modified 5'-cap analog therefore allows *in vitro* and *in vivo* studies under conditions that allow for remethylation.

The modified 5'-cap analogs provide for applications in basic research to study the function of proteins with spatio-temporal control, such as in developmental biology. As mRNA is usable as a therapeutic agent the modified 5'-cap analogs, particularly the N7G-modified 5'-cap analog, also provide for therapeutic applications.

The term "5'-cap" as used herein refers to a cap structure that is attached at the 5'-end to the first transcribed nucleotide of an mRNA chain. The 5'-cap comprises a 7-methylguanosine attached via a oligophosphate group such as a 5'-5' triphosphate bridge, or in some cases a methyl phosphate or methyleneoligophosphate group, to the mRNA.

The term "5'-cap analog" as used herein refers to a functionalized cap structure that can be incorporated into RNA at the 5'-end, such as by *in vitro* transcription (IVT), analogous to the natural cap. The modified 5'-cap analogs can be enzymatically incorporated at the 5'-end of an mRNA, such as in a cell or in an organism. The 5' cap modification will block translation of a respective mRNA until a radiation-triggered removal. Upon irradiation of the modified 5'-cap analog the photocleavable coumarin group and unmethylated cap are released. The 5'-cap analog can have a cap 0, cap 1 or cap 2 structure.

The term "photocleavable" refers to a moiety or chemical group that can be removed via irradiation. Such chemical groups also are denoted photoremovable or photosensitive. Such a group can provide a function in regard to the structure it is attached to, such as functioning as hiding or hindering ("caging") a biological activity of the structure it is attached to. In regard to the 5'-cap the photocleavable coumarin group hinders the functionality of a natural 5'-cap and the photocleavable coumarin group may be related to as a photo-caging group. As used herein, the photocleavable coumarin group may also be referred to as a photocaging group.

It was found that a coumarin group can be cleaved residue-free under biologically compatible conditions from the positively charged nitrogen at the N7-position of guanosine and the N1-position of adenosine. This allows to provide photo-controllable nucleoside and nucleotide analogs comprising a photocleavable coumarin group at a positively charged nitrogen of nucleobases, such as the N7-position of guanosine, the N1-position of adenosine, or the N3-position cytidine.

According to a further aspect is provided a photocleavable nucleoside or nucleotide analog or salt thereof, wherein the analog is a nucleoside or nucleotide analog according to formulas (IV), (V) or (VI) as given as follows and comprises a photocleavable group PC-Cou at the N7-position of guanosine, at the N1-position of adenosine, or at the N3-position of cytidine: 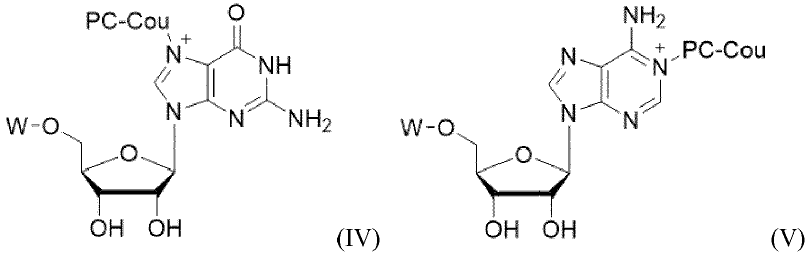 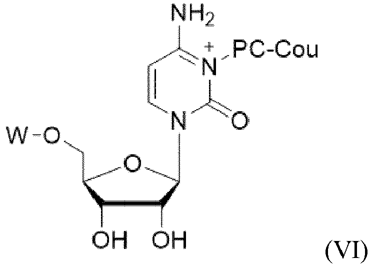 wherein:
- W: is H or a modified or unmodified mono-phosphate, di-phosphate, or tri-phosphate group, and
- PC-Cou: is a photocleavable coumarin group.

In embodiments, W is hydrogen. If provided to cells, the photocaged nucleosides can be phosphorylated *in vivo* or *in vitro* and are usable in the photoactivatable labelling of RNA or DNA.

In embodiments, W is a mono-, di-, or tri-phosphate group. The group W may be unmodified or modified, such as with naturally occurring or not naturally occurring groups such as NH, O, CH₂, S, Se or BH₃. In embodiments, the group W is an unmodified mono-, di-, or tri-phosphate group, preferably an unmodified mono-phosphate or tri-phosphate.

The photocleavable nucleoside or nucleotide analog and the photocleavable 5'-cap analog comprise a photocleavable coumarin group denoted "PC-Cou". Preferably, PC-Cou is a photocleavable (coumarin-4-yl)methyl compound. In embodiments, PC-Cou is a photocleavable coumarin selected from the group of (coumarin-4-yl)methyl compounds according to formulas (P1) to (P23) as given as follows: 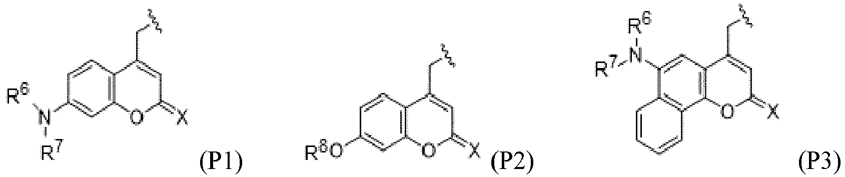 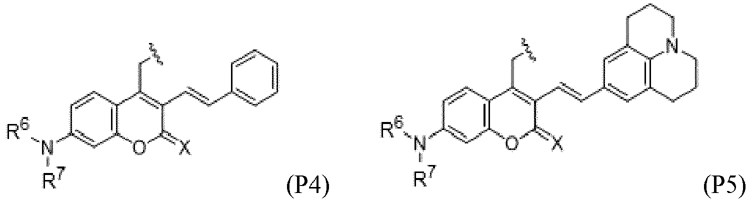 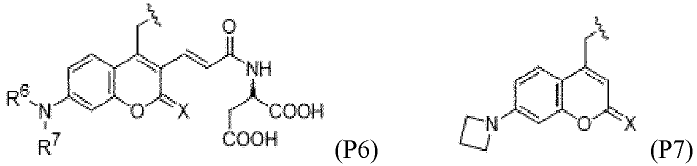 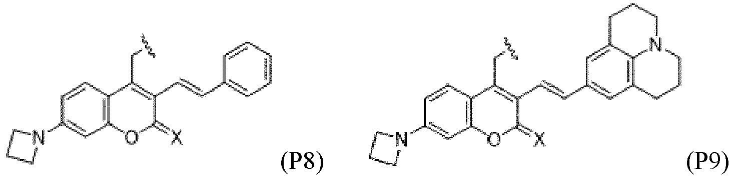 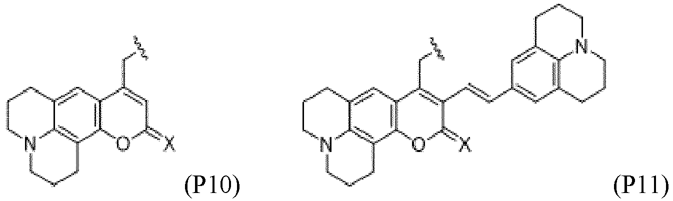 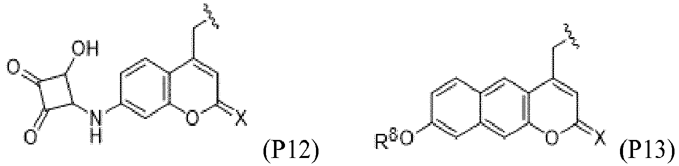 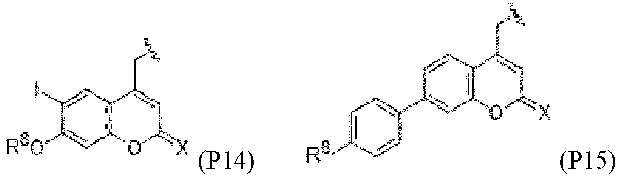 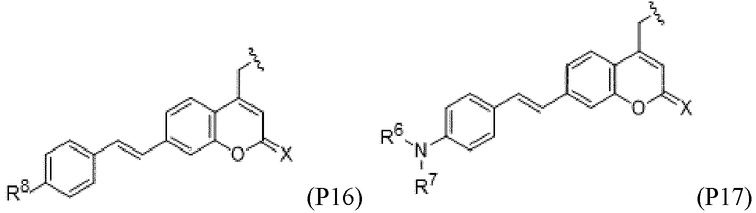 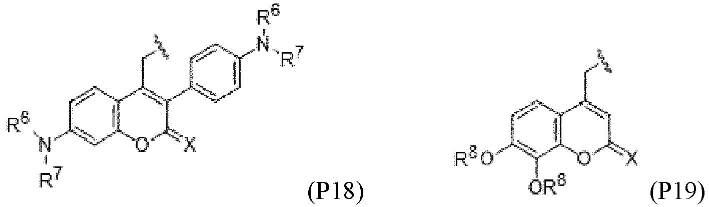 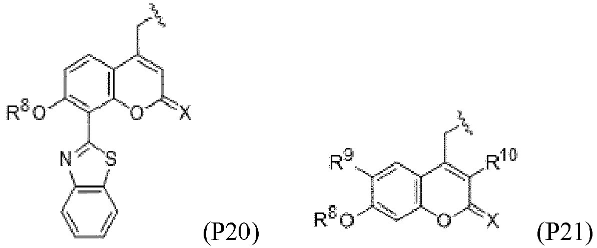 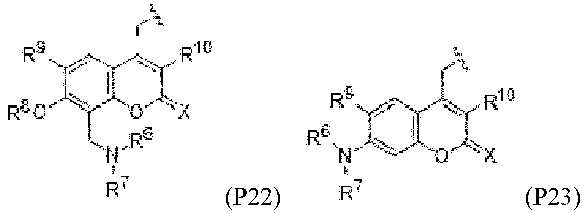 wherein:
- R⁶, R⁷: are independently selected from the group of H, C₁-C₁₀ alkyl, C₃-C₇ cycloalkyl, and 2-propynyl;
- R⁸: is selected from the group of H, C₁-C₁₀ alkyl, C₁-C₁₀ COOH, and C₁-C₁₀ alkoxy;
- R⁹, R¹⁰: are independently selected from the group of H, I or Br; and
- X: is selected from the group of O, S and Se.

Unless specifically stated otherwise, compounds, groups or substituents denoted with Arabic numerals differ from compounds, groups or substituents denoted with Roman numerals or a combined naming of numerals and letters, that is, compounds, groups or substituents are different compounds, groups or substituents.

The coumarin groups show low or tolerable toxicity and thus are usable *in vitro* and *in vivo.* Further, the (coumarin-4-yl)methyl compounds, provide advantageous photoremovability upon irradiation in a range from 365 nm to 625 nm.

The term "alkyl" as used herein is to be understood as meaning straight-chain or branched alkyl groups. The term "C₁-C₁₀-alkyl" as used herein refers to alkyl groups having 1 to 10 carbon atoms. Preferred are C₁-C₆-Alkyl groups, which may be selected from the group comprising methyl, ethyl, propyl, butyl, pentyl and hexyl. Preferred are C₁-C₃-alkyl groups, particularly methyl and ethyl.

The term "C₁-C₁₀-alkoxy" is to be understood as meaning a C₁-C₁₀-alkyl bonded to oxygen. Preferred are C₁-C₆-alkoxy groups, particularly methoxy and ethoxy.

The C₃-C₇ cycloalkyl group preferably is a C₅-C₆ cycloalkyl group, particularly cyclopentyl or cyclohexyl.

Preferred carboxy groups "C₁-C₁₀-COOH" are C₁-C₅-COOH or C₁-C₃-COOH groups, particularly ethanoic acid.

In embodiments, R⁶ and R⁷ are independently selected from the group of H, methyl, ethyl, C₅-C₆ cycloalkyl, and 2-propynyl. In embodiments, R⁸ is selected from the group of H, methyl, CH₂-COOH and methoxy. In embodiments, R⁹ and R¹⁰ are independently hydrogen.
In embodiments, X is selected from O and S.

In embodiments, R⁶, R⁷ are independently selected from the group of H, methyl, ethyl, C₅-C₆ cycloalkyl, and 2-propynyl; R⁸ is selected from the group of H, methyl, CH₂-COOH and methoxy; R⁹, R¹⁰ are independently selected from the group of H, I or Br; and X is selected from the group of O and S.

In embodiments, PC-Cou is a photocleavable (coumarin-4-yl)methyl compound according to formulas (P24) to (P32) as given as follows

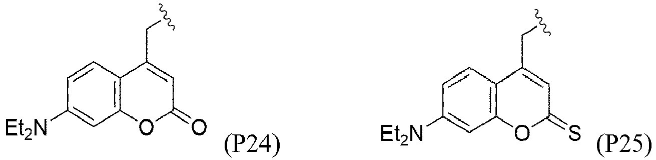

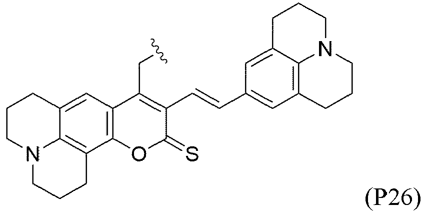

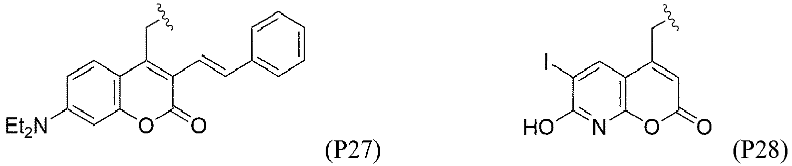

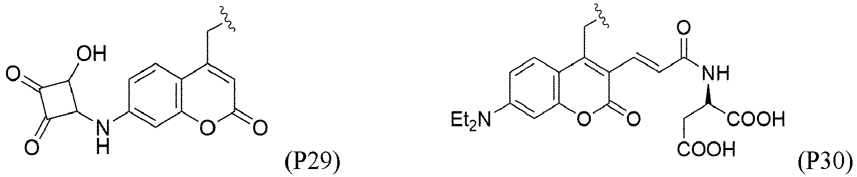

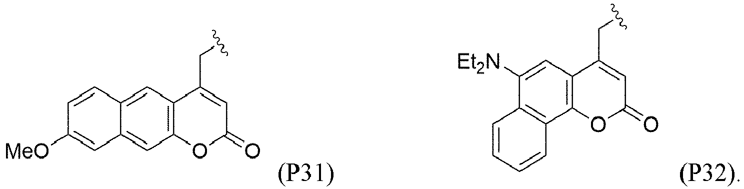

In preferred embodiments, PC-Cou is the photocleavable [7-(diethylamino)coumarin-4-yl]methyl according to formula (P24) as given as follows: 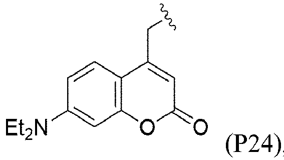 , wherein Et is ethyl.

The photocleavable (coumarin-4-yl)methyl compound of formula (P24) has an absorption maxima at 450 nm.

The groups B¹ and B² each represent a modified or unmodified nucleobase. As used herein, the term "nucleobase" includes all forms of nucleoside bases found in nature. Base rings most commonly found in naturally occurring nucleosides are purine and pyrimidine rings. Naturally occurring purine rings include, for example, adenine, guanine, N⁷-methylguanine and N⁶- methyladenine. Naturally occurring pyrimidine rings include, for example, cytosine, thymine, 5-methylcytosine, N1-methyl-pseudouracil, pseudouracil. The nucleobases may be modified, such as by substitution with protecting groups and/or halogen such as fluor and include, for example, 2'-deoxy-2'-fluorouridine and 5- fluorouridine. The term modified nucleobase as used herein includes halogen-substituted purines such as 6-fluoropurine, halogen-substituted pyrimidines, N⁶-ethyladenine, N(alkyl)-cytosines or 5-methylcytosine. Unmodified nucleobases B¹ and B² independently may be selected from guanine, cytosine, thymine, uracil, adenine, N⁷-methylguanine or N⁶-methyladenine. In embodiments, B¹ and B² independently are a natural nucleobase selected from guanine or adenine. In embodiments, B¹ and B² independently are guanine.

The group U is a modified or unmodified oligophosphate or methyleneoligophosphate group. The group U may be an oligophosphate or methyleneoligophosphate group modified with naturally occurring or not naturally occurring groups such as NH, O, CH₂, S, Se or BH₃. In embodiments, U is an oligophosphate or methyleneoligophosphate group selected from the group of compounds according to formulas (U1) to (U5) as given as follows or salts thereof: 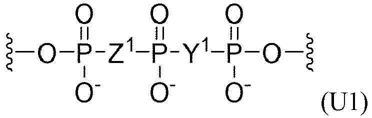 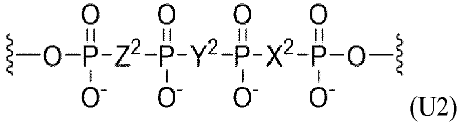 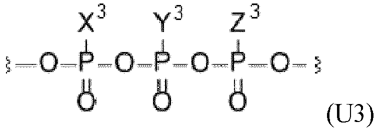 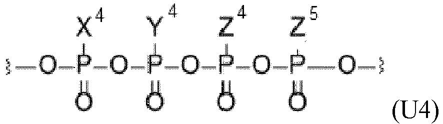 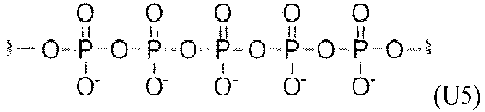 wherein:
- Y¹, Z¹: are independently selected from the group of O, NH and CH₂;
- X², Y², Z²: are independently selected from the group of O, NH and CH₂;

- X³, Y³, Z³: are independently selected from the group of O, S, Se and BH₃;
- X⁴, Y⁴, Z⁴, Z⁵: are independently O or S.

The group U may be a di- or triphosphate or a di- or triphosphonate group modified with one or more sulfur atoms. In embodiments, U is a modified oligophosphate or methyleneoligophosphate group selected from the group of compounds according to formulas (U6) to (U16) as given as follows or salts thereof:

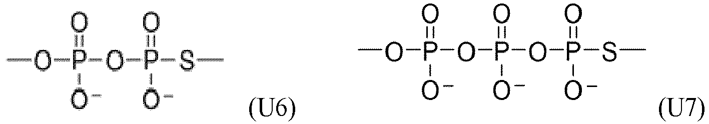

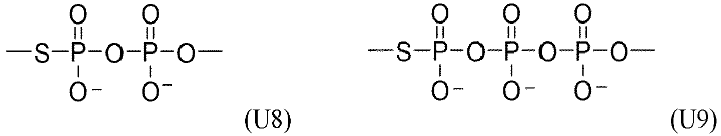

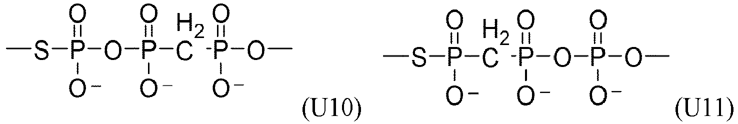

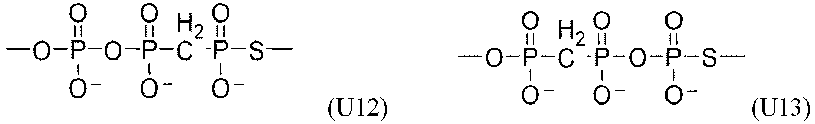

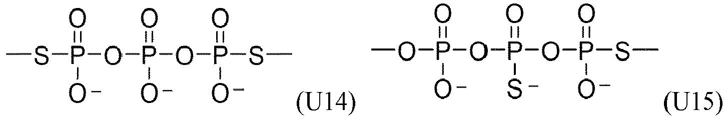

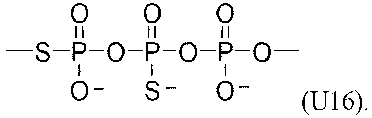

In embodiments, particularly in embodiments of the 5'-cap analog of formula (I), U is a triphosphate or methylenetriphosphate compound according to formula (U1) wherein Y¹ and Z¹ both are O, or one of Y¹ and Z¹ is O and the other CH₂.

In other embodiments of the 5'-cap analog of formulas (I) and (II), U is a triphosphate according to formula (U1) wherein Y¹ and Z¹ both are O, or a tetraphosphate according to formula (U2) wherein X², Y² and Z² are O, or a pentaphosphate according to formula (U5). In other embodiments, dinucleoside tetraphosphates (Np₄Ns), preferably Ap4A, Gp4A, Cp4A, and Up4A, according to formula (I) and (II) are preferred.

The 5'-cap analog of formula (I) comprises a photocaged guanosine. In embodiments of the 5'-cap analog of formula (I), the groups R¹, R² independently may be H, OH or OCH₃. Preferably, the groups R¹ and R² are OH or OCH₃, more preferred the groups R¹ and R² are OH.

Referring to the photocleavable guanosine 5'-cap analog of formula (I), in an embodiment for a cap 0 analog, R³ and R⁵ are hydrogen. In an embodiment for a cap 1 analog, R³ is hydrogen and R⁵ is CH₃. In an embodiment for a cap 2 analog, R³ and R⁵ are CH₃.

The group R³ may be H or CH₃. The photocleavable 5'-cap analog may carry a terminal 3'-OH group that is a valid substrate for RNA polymerase. In other embodiments, the photocleavable 5'-cap analog may carry a terminal 3'-OCH₃ group.

The group R⁴ may be hydrogen or an element according to formula (III) or a salt thereof wherein R⁵ is H or CH₃:

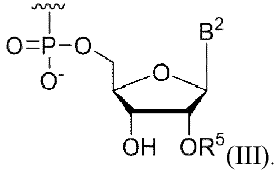

In embodiments R⁴ is an element according to formula (III). The group R⁵ may be hydrogen. In embodiments, the photocleavable 5'-cap analog may have a structure according to formula (Ia) as given as follows or salts thereof wherein R⁵ is H or CH₃:

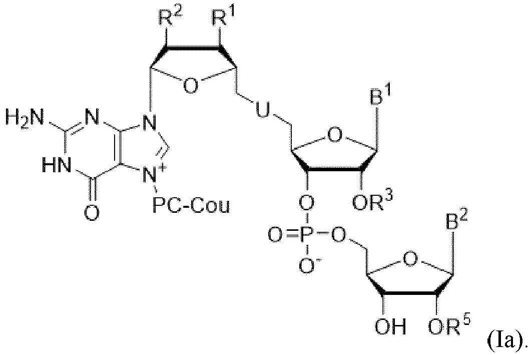

The group R⁵ may be hydrogen.

In these embodiments, B¹ may be selected from guanine, cytosine, thymine, uracil, adenine and N⁶-methyladenine, preferably from guanine and adenine. Preferably, B² may be selected from guanine, cytosine, thymine, uracil, adenine or N⁶-methyladenine, preferably from guanine, uracil, and adenine. In preferred embodiments, B¹ is adenine and B² is guanine, or B¹ is adenine and B² is uracil, or B¹ and B² are guanine.

Preferably R⁴ is hydrogen. In such embodiments, the photocleavable 5'-cap analog may have a structure according to formula (Ib) as given as follows or salts thereof:

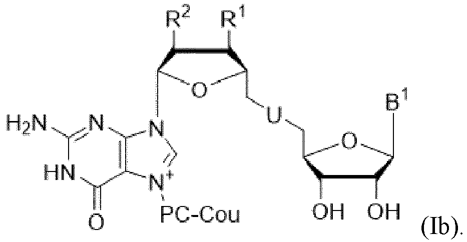

In these embodiments, B¹ may be a natural nucleobase selected from guanine, cytosine, thymine, uracil, adenine and N⁶-methyladenine. Preferably, B¹ may be guanine or adenine.

Referring to the compounds according to formulas (Ia) and (Ib), the elements PC-Cou, B¹, R¹, R², R³ independently may be selected as discussed above.

In embodiments, the photocleavable 5'-cap analog has a structure according to formula (VII) as given as follows or salts thereof: 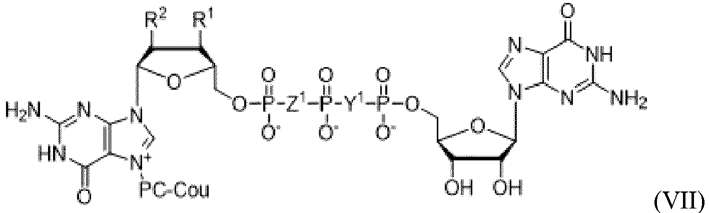 wherein R¹, R² are independently H, OH or OCH₃; and Y¹, Z¹ are independently selected from the group of O, NH and CH₂. Exemplary embodiments of photocleavable 5'-cap analogs according to formula (VII) are summarized in table 1.

**Table 1: Exemplary embodiments of photocleavable 5'-cap analogs according to formula (VII)**

| Abbreviation | R¹ | R² | Y¹ | Z¹ |
|---|---|---|---|---|
| PC-Cou⁷GpppG | OH | OH | O | O |
| PC-Cou⁷m^{3'-O}GpppG | OCH₃ | OH | O | O |
| PC-Cou⁷m^{2'-O}GpppG | OH | OCH₃ | O | O |
| PC-Cou⁷GppCH₂pG | OH | OH | CH₂ | O |
| PC-Cou⁷GpCH₂ppG | OH | OH | O | CH₂ |
| PC-Cou⁷m^{3'-O}GppCH₂pG | OCH₃ | OH | CH₂ | O |
| PC-Cou⁷m^{3'-O} GpCH₂ppG | OCH₃ | OH | O | CH₂ |
| PC-Cou⁷m^{2'-O}GppCH₂pG | OH | OCH₃ | CH₂ | O |
| PC-Cou⁷GppNHpG | OH | OH | NH | O |
| PC-Cou⁷GpNHppG | OH | OH | O | NH |
| PC-Cou⁷m^{2'-O}GppNHpG | OH | OCH₃ | NH | O |
| PC-Cou⁷m^{2'-O}GpNHppG | OH | OCH₃ | O | NH |

A preferred embodiment is the photocleavable 5'-cap analog PC-Cou⁷GpppG according to formula (VIIa) as given as follows or salts thereof:

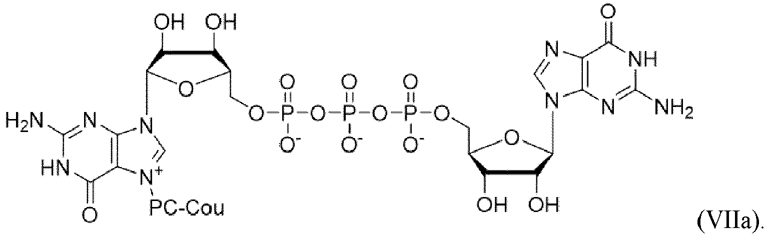

In other embodiments, the photocleavable 5'-cap analog has a structure according to formula (VIII) as given as follows or salts thereof: 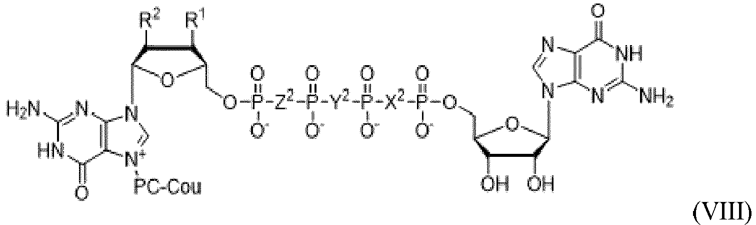 wherein R¹, R² are independently selected from the group of OH or OCH₃; and X², Y², Z² are independently selected from the group of O, NH and CH₂. Exemplary embodiments of photocleavable 5'-cap analogs according to formula (VIII) are summarized in table 2.

**Table 2: Exemplary embodiments of photocleavable 5'-cap analogs according to formula (VIII)**

| Abbreviation | R¹ | R² | X² | Y² | Z² |
|---|---|---|---|---|---|
| PC-Cou⁷m^{2'-O}Gp₄G | OH | OCH₃ | O | O | O |
| PC-Cou⁷2'dGp₄G | OH | H | O | O | O |
| PC-Cou⁷GpppCH₂pG | OH | OH | CH₂ | O | O |
| PC-Cou⁷GppCH₂ppG | OH | OH | O | CH₂ | O |
| PC-Cou⁷GpCH₂pppG | OH | OH | O | O | CH₂ |
| PC-Cou⁷m^{2'-O}GpppCH₂pG | OH | OCH₃ | CH₂ | O | O |
| PC-Cou⁷m^{2'-O}GppCH₂pG | OH | OCH₃ | O | CH₂ | O |
| PC-Cou⁷m^{2'-O}GpCH₂pppG | OH | OCH₃ | O | O | CH₂ |
| PC-Cou⁷GpppNHpG | OH | OH | NH | O | O |
| PC-Cou⁷m^{2'-O}GpppNHpG | OH | OCH₃ | NH | O | O |
| PC-Cou⁷GpNHpppG | OH | OH | O | O | NH |
| PC-Cou⁷m^{2'-O}GpNHpppG | OH | OCH₃ | O | O | NH |

In other embodiments, the photocleavable 5'-cap analog has a structure according to formula (IX) as given as follows or salts thereof: 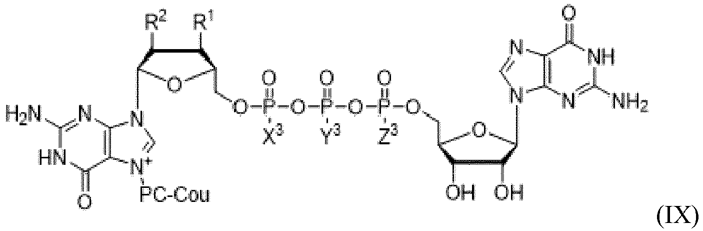 wherein R¹, R² are independently OH or OCH₃; and X³, Y³, Z³ are independently selected from the group of O, S, Se and BH₃. Exemplary embodiments of photocleavable 5'-cap analogs according to formula (IX) are summarized in table 3.

**Table 3: Exemplary embodiments of photocleavable 5'-cap analogs according to formula (IX)**

| Abbreviation | R¹ | R² | X³ | Y³ | Z³ |
|---|---|---|---|---|---|
| PC-Cou⁷Gppp_{S}G | OH | OH | O | O | S |
| PC-Cou⁷Gpp_{S}pG | OH | OH | O | S | O |
| PC-Cou⁷Gp_{S}ppG | OH | OH | S | O | O |
| PC-Cou⁷m^{2'-O}Gppp_{S}G | OH | OCH₃ | O | O | S |
| PC-Cou7m^{2'-O}Gpp_{S}pG | OH | OCH₃ | O | S | O |
| PC-Cou7m^{2'-O}Gp_{S}ppG | OH | OCH₃ | S | O | O |
| PC-Cou⁷m^{2'-O}Gpp_{Se}pG | OH | OCH₃ | O | Se | O |
| PC-Cou⁷Gppp_{BH3}G | OH | OH | O | O | BH₃ |
| PC-Cou⁷Gpp_{BH3}pG | OH | OH | O | BH₃ | O |
| PC-Cou⁷Gp_{BH3}ppG | OH | OH | BH₃ | O | O |
| PC-Cou⁷m^{2'-O}Gpp_{PBH3}G | OH | OCH₃ | O | O | BH₃ |
| PC-Cou⁷m^{2'-O}Gp_{PBH3}pG | OH | OCH₃ | O | BH₃ | O |

In further embodiments, the photocleavable 5'-cap analog has a structure according to formula (X) as given as follows or salts thereof: 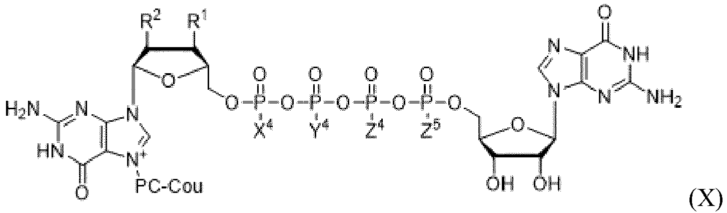 wherein R¹, R² are independently OH or OCH₃ and X⁴, Y⁴, Z⁴, Z⁵ are independently O or S. Exemplary embodiments of photocleavable 5'-cap analogs according to formula (X) are summarized in table 4.

**Table 4: Exemplary embodiments of photocleavable 5'-cap analogs according to formula (X)**

| Abbreviation | R¹ | R² | X⁴ | Y⁴ | Z⁴ | Z⁵ |
|---|---|---|---|---|---|---|
| PC-Cou⁷m^{2'-O}Gpppp_{S}G | OH | OCH₃ | O | O | O | S |
| PC-Cou⁷m^{2'-O}Gppp_{S}pG | OH | OCH₃ | O | O | S | O |
| PC-Cou⁷m^{2'-O}Gpp_{S}ppG | OH | OCH₃ | O | S | O | O |
| PC-Cou⁷m^{2'-O}Gp_{S}pppG | OH | OCH₃ | S | O | O | O |

In further embodiments, tetraphosphate and pentaphosphate photocleavable 5'-caps having a PC-CouN7G modification are selected from PC-Cou⁷Gp₄A, m⁶Ap₄PC-Cou⁷G and m⁶Ap₅PC-Cou⁷G.

In an embodiment, the photocleavable 5'-cap analog has a structure according to formula (2) as given as follows or salts thereof, wherein Et is ethyl:

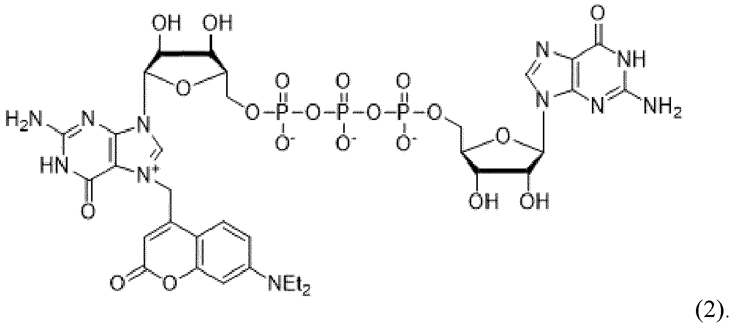

The 5'-cap analog of formula (II) comprises a photocaged adenosine. 5'-Cap analogs comprising adenosine can be incorporated as a transcription start nucleotide in eukaryotic RNA and also in bacterial RNA.

Unlike caps such as GpppG, a cap GpppA provides two different orientations of the molecule as shown below for Gppp(PC-Cou)¹A of formula (XI) and (PC-Cou)¹ApppB of formula (XII):

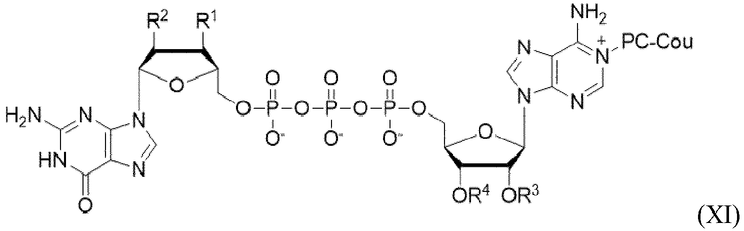

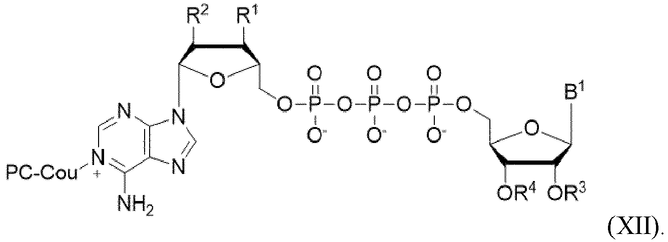

In both cases the respective cap can be used in different ways for downstream applications depending on the DNA template.

In one embodiment of the 5'-cap analog of formula (II), the groups A¹ and A² are R¹ and R² and the groups A³ and A⁴ are OR³ and OR⁴. In another embodiment of the 5'-cap analog of formula (II), the groups A¹ and A² are OR⁴ and OR³ and the groups A³ and A⁴ are R² and R¹. This embodiment allows for the group B¹ being N⁷-methylguanosine (m7G) that the 5'-cap analog can be incorporated as m⁷Gppp(PC-Cou)¹A.

In a preferred embodiment, the 5'-cap analog of formula (II) has a structure according to formula (XIa) as given as follows or salts thereof:

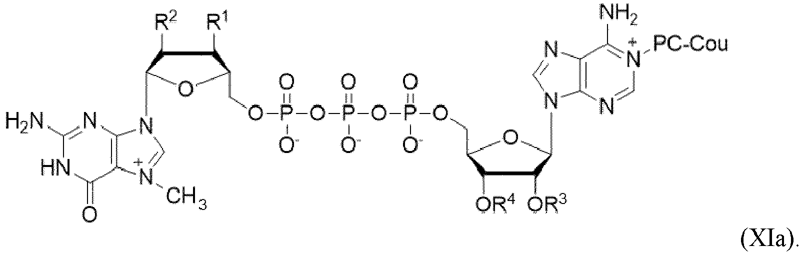

Also in the embodiments of the 5'-cap analog of formula (II), the groups R¹, R² independently may be H, OH or OCH₃. Preferably, the groups R¹ and R² are OH or OCH₃, more preferred the groups R¹ and R² are OH.

Also referring to a photocleavable 5'-cap analog of formula (II), in an embodiment for a cap 0 analog, R³ and R⁵ are hydrogen. In an embodiment for a cap 1 analog, R³ is hydrogen and R⁵ is CH₃. In an embodiment for a cap 2 analog, R³ and R⁵ are CH₃.

The group R³ may be H or CH₃. The photocleavable 5'-cap analog may carry a terminal 3'-OH group that is a valid substrate for RNA polymerase. In other embodiments, the photocleavable 5'-cap analog may carry a terminal 3'-OCH₃ group.

In embodiments R⁴ is an element according to formula (III). In embodiments, the photocleavable 5'-cap analog may have a structure according to formula (IIa) as given as follows or salts thereof wherein R⁵ is H or CH₃:

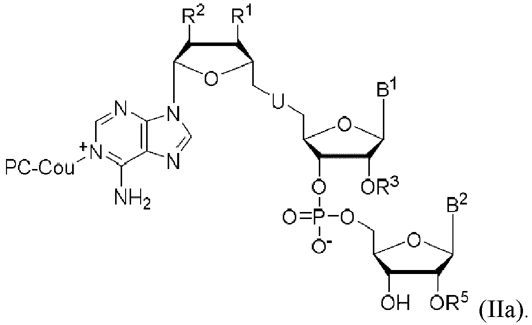

The group R⁵ may be hydrogen.

In these embodiments, B¹ may be selected from guanine, cytosine, thymine, uracil, adenine, N⁷-methylguanine and N⁶-methyladenine, preferably from guanine, N⁷-methylguanine and adenine. Preferably, B² may be selected from guanine, cytosine, thymine, uracil, adenine or N⁶-methyladenine, preferably from guanine, uracil, and adenine. In preferred embodiments, B¹ is adenine and B² is guanine, or B¹ is adenine and B² is uracil, or B¹ and B² are guanine.

Preferably R⁴ is hydrogen. In such embodiments, the photocleavable 5'-cap analog may have a structure according to formula (IIb) as given as follows or salts thereof:

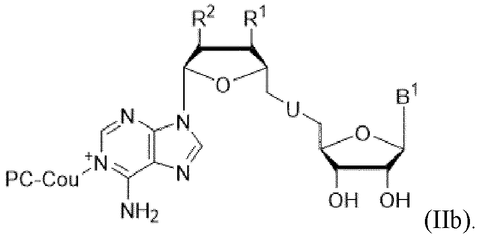

In these embodiments, B¹ may be a natural nucleobase selected from guanine, cytosine, thymine, uracil, adenine, N⁷-methylguanine and N⁶-methyladenine. Preferably B¹ may be guanine, N⁷-methylguanine or adenine.

Referring to the compounds according to formulas (IIa) and (IIb), the elements PC-Cou, B¹, R¹, R², R³ independently may be selected as discussed above.

In embodiments, the photocleavable 5'-cap analog has a structure according to formula (XIII) as given as follows or salts thereof: 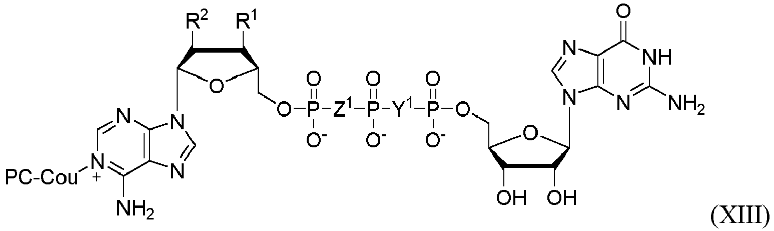 wherein R¹, R² are independently H, OH or OCH₃; and Y¹, Z¹ are independently selected from the group of O, NH and CH₂. Exemplary embodiments of photocleavable 5'-cap analogs according to formula (XIII) are summarized in table 5.

**Table 5: Exemplary embodiments of photocleavable 5'-cap analogs according to formula (XIII)**

| Abbreviation | R¹ | R² | Y¹ | Z¹ |
|---|---|---|---|---|
| PC-Cou¹ApppG | OH | OH | O | O |
| PC-Cou⁷m^{3'-O}ApppG | OCH₃ | OH | O | O |
| PC-Cou⁷m^{2'-O}ApppG | OH | OCH₃ | O | O |
| PC-Cou¹AppCH₂pG | OH | OH | CH₂ | O |
| PC-Cou¹ApCH₂ppG | OH | OH | O | CH₂ |
| PC-Cou¹m^{3'-O}AppCH₂pG | OCH₃ | OH | CH₂ | O |
| PC-Cou¹m^{3'-O}ApCH₂ppG | OCH₃ | OH | O | CH₂ |
| PC-Cou¹m^{2'-O}AppCH₂pG | OH | OCH₃ | CH₂ | O |
| PC-Cou¹AppNHpG | OH | OH | NH | O |
| PC-Cou¹ApNHppG | OH | OH | O | NH |
| PC-Cou¹m^{2'-O}AppNHpG | OH | OCH₃ | NH | O |
| PC-Cou¹m^{2'-O}ApNHppG | OH | OCH₃ | O | NH |

In other embodiments, the photocleavable 5'-cap analog has a structure according to formula (XIV) as given as follows or salts thereof: 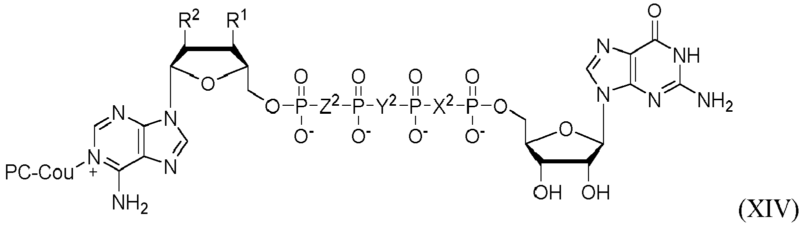 wherein R¹, R² are independently selected from the group of OH or OCH₃; and X², Y², Z² are independently selected from the group of O, NH and CH₂. Exemplary embodiments of photocleavable 5'-cap analogs according to formula (XIV) are summarized in table 6.

**Table 6: Exemplary embodiments of photocleavable 5'-cap analogs according to formula (XIV)**

| Abbreviation | R¹ | R² | X² | Y² | Z² |
|---|---|---|---|---|---|
| PC-Cou¹m^{2'-O}Ap₄G | OH | OCH₃ | O | O | O |
| PC-Cou¹2'dAp₄G | OH | H | O | O | O |
| PC-Cou¹ApppCH₂pG | OH | OH | CH₂ | O | O |
| PC-Cou¹AppCH₂ppG | OH | OH | O | CH₂ | O |
| PC-Cou¹ApCH₂pppG | OH | OH | O | O | CH₂ |
| PC-Cou¹m^{2'-O}ApppCH₂pG | OH | OCH₃ | CH₂ | O | O |
| PC-Cou¹m^{2'-O}AppCH₂pG | OH | OCH₃ | O | CH₂ | O |
| PC-Cou¹m^{2'-O}ApCH₂pppG | OH | OCH₃ | O | O | CH₂ |
| PC-Cou¹ApppNHpG | OH | OH | NH | O | O |
| PC-Cou¹m^{2'-O}ApppNHpG | OH | OCH₃ | NH | O | O |
| PC-Cou¹ApNHpppG | OH | OH | O | O | NH |
| PC-Cou¹m^{2'-O}ApNHpppG | OH | OCH₃ | O | O | NH |

In other embodiments, the photocleavable 5'-cap analog has a structure according to formula (XV) as given as follows or salts thereof: 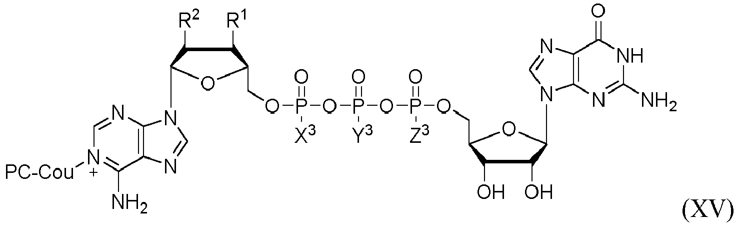 wherein R¹, R² are independently OH or OCH₃; and X³, Y³, Z³ are independently selected from the group of O, S, Se and BH₃. Exemplary embodiments of photocleavable 5'-cap analogs according to formula (XV) are summarized in table 7.

**Table 7: Exemplary embodiments of photocleavable 5'-cap analogs according to formula (XV)**

| Abbreviation | R¹ | R² | X³ | Y³ | Z³ |
|---|---|---|---|---|---|
| PC-Cou¹Appp_{S}G | OH | OH | O | O | S |
| PC-Cou¹App_{S}pG | OH | OH | O | S | O |
| PC-Cou¹Ap_{S}ppG | OH | OH | S | O | O |
| PC-Cou¹m^{2'-O}Appp_{S}G | OH | OCH₃ | O | O | S |
| PC-Cou¹m^{2'-O}App_{S}pG | OH | OCH₃ | O | S | O |
| PC-Cou¹m^{2'-O}Ap_{S}ppG | OH | OCH₃ | S | O | O |
| PC-Cou¹m^{2'-O}App_{Se}pG | OH | OCH₃ | O | Se | O |
| PC-Cou¹Appp_{BH3}G | OH | OH | O | O | BH₃ |
| PC-Cou¹App_{BH3}pG | OH | OH | O | BH₃ | O |
| PC-Cou¹Ap_{BH3}ppG | OH | OH | BH₃ | O | O |
| PC-Cou¹m^{2'-O}Appp_{BH3}G | OH | OCH₃ | O | O | BH₃ |
| PC-Cou¹m^{2'-O}App_{BH3}pG | OH | OCH₃ | O | BH₃ | O |

In further embodiments, the photocleavable 5'-cap analog has a structure according to formula (XVI) as given as follows or salts thereof: 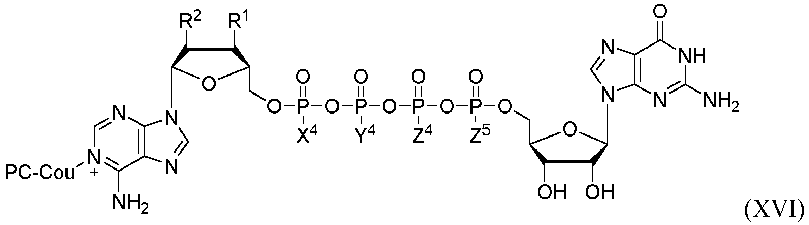 wherein R¹, R² are independently OH or OCH₃ and X⁴, Y⁴, Z⁴, Z⁵ are independently O or S. Exemplary embodiments of photocleavable 5'-cap analogs according to formula (XVI) are summarized in table 8.

**Table 8: Exemplary embodiments of photocleavable 5'-cap analogs according to formula (XVI)**

| Abbreviation | R¹ | R² | X⁴ | Y⁴ | Z⁴ | Z⁵ |
|---|---|---|---|---|---|---|
| PC-Cou¹m^{2'-O}Apppp_{S}G | OH | OCH₃ | O | O | O | S |
| PC-Cou¹m^{2'-O}Appp_{S}pG | OH | OCH₃ | O | O | S | O |
| PC-Cou¹m^{2'-O}App_{S}ppG | OH | OCH₃ | O | S | O | O |
| PC-Cou¹m^{2'-O}Ap_{S}pppG | OH | OCH₃ | S | O | O | O |

A preferred embodiment is a photocleavable 5'-cap analog PC-Cou¹ApppA according to formula (XVII) as given as follows or salts thereof:

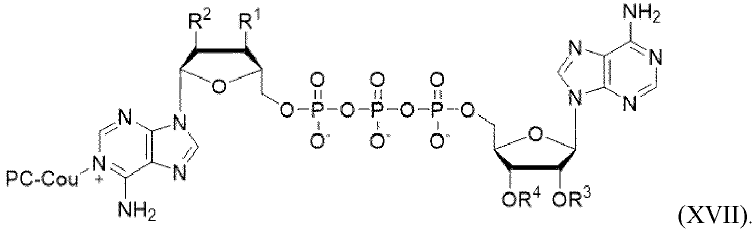

A further preferred embodiment is a photocleavable 5'-cap analog Appp(PC-Cou)¹A according to formula (XVIII) as given as follows or salts thereof:

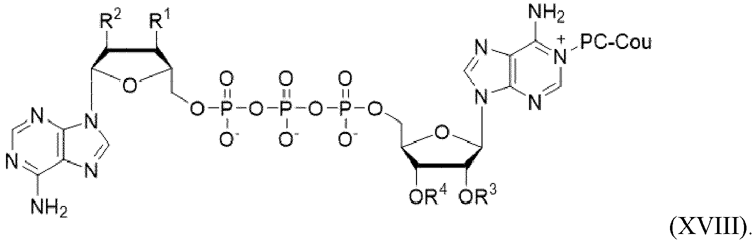

Another preferred embodiment is a photocleavable 5'-cap analog PC-Cou¹AppppG according to formula (XIX) as given as follows or salts thereof:

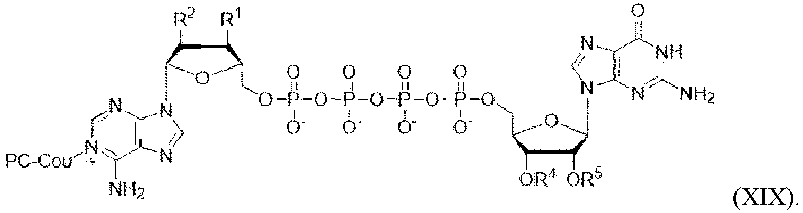

A further preferred embodiment is a photocleavable 5'-cap analog Gpppp(PC-Cou¹)A according to formula (XX) as given as follows or salts thereof:

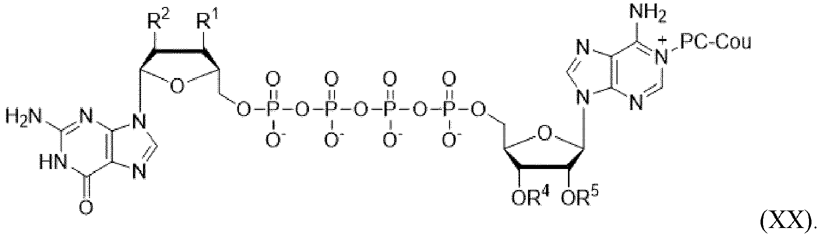

A further preferred embodiment is a photocleavable 5'-cap analog PC-Cou¹ApppppG according to formula (XXI) as given as follows or salts thereof:

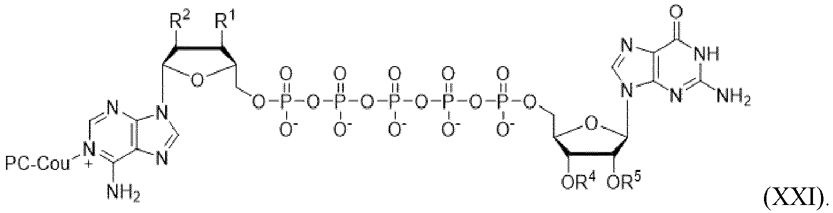

Another preferred embodiment is a photocleavable 5'-cap analog Gppppp(PC-Cou¹)A according to formula (XXII) as given as follows or salts thereof:

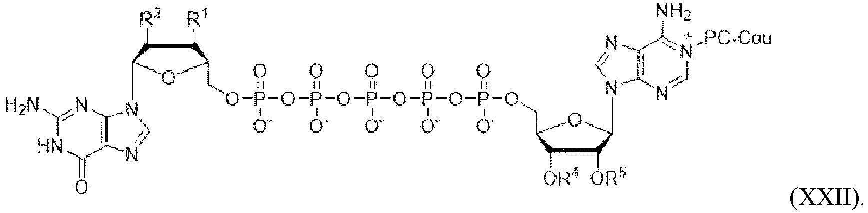

The triphosphate caps of formulas (XVII) and (XVIII), the tetraphosphate caps of formulas (XIX) and (XX), and the pentaphosphate caps of formulas (XXI) and (XXII) are usable for bacterial caps.

In further embodiments, photocleavable triphosphate 5'-caps having a PC-CouN1A modification are selected from PC-Cou¹Ap₃A, PC-Cou¹Ap₃G, Gp₃PC-Cou¹A, m⁷Gp₃PC-Cou¹A, m⁶Ap₃PC-Cou¹A, and m⁶PC-Cou¹Ap₃A.

In further embodiments, photocleavable tetraphosphate 5'-caps having a PC-CouN1A modification are selected from PC-Cou¹Ap₄A, PC-Cou¹Ap₄m⁶A, Gp₄PC-Cou¹A, m⁷Gp₄PC-Cou¹A, m⁶PC-Cou¹Ap₄G, Cp₄PC-Cou¹A and Up₄PC-Cou¹A.

In further embodiments, photocleavable pentaphosphate 5'-caps having a PC-CouN1A modification are selected from PC-Cou¹Ap₅A, PC-Cou¹Ap₅m⁶A, m⁶Ap₅PC-Cou¹A, PC-Cou¹Ap₅m⁶A, PC-Cou¹Ap₅A, PC-Cou¹Ap₅m⁶A, PC-Cou¹Ap₅G and m⁶PC-Cou¹Ap₅G.

In embodiments, the photocleavable 5'-cap analog has a structure according to formula (4) as given as follows or salts thereof:

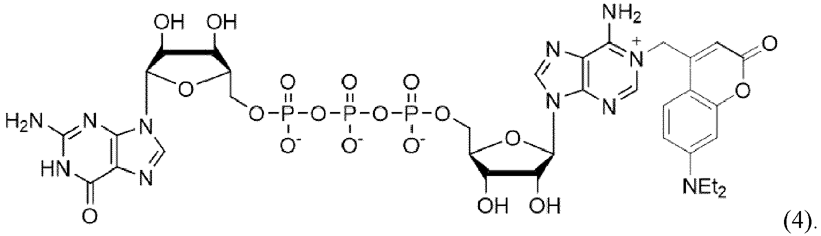

In embodiments, the photocleavable nucleoside or nucleotide analog has a structure according to formula (1) as given as follows or salts thereof, wherein Et is ethyl:

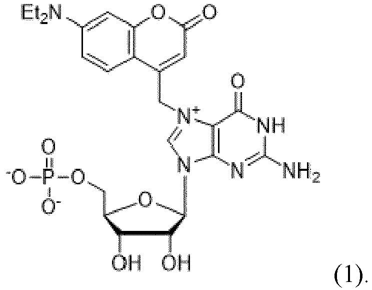

In other embodiments, the photocleavable nucleoside or nucleotide analog has a structure according to formula (3) as given as follows or salts thereof, wherein Et is ethyl:

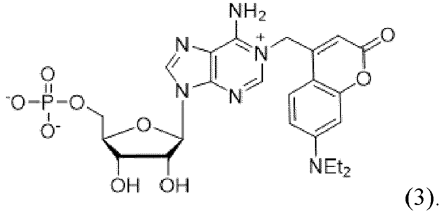

The photocleavable 5'-cap analogs and nucleoside or nucleotide analogs may be provided in form of its salt. Particularly for use in cells or biological assays, the photocleavable 5'-cap analog and nucleoside or nucleotide analog may be provided in form of a pharmaceutically acceptable salt thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferred are potassium and sodium salts. In preferred embodiments, the photocleavable 5'-cap analog or nucleoside or nucleotide analog is provided in form of a sodium salt.

The photocleavable 5'-cap analog may be used as a tool for studying the function of proteins. Further, the photocleavable 5'-cap analog is usable as therapeutic tool or as a medicament. According to an aspect, the photocleavable 5'-cap analog is usable for inhibiting cap-binding proteins, such as the eukaryotic initiation factor (eIF4E), or cap-degrading enzymes. The inhibition of cap-binding proteins can be annulled via photocleaving of the 5'-cap analog. A cap-binding protein is, for example, eukaryotic translation initiation factor 4 (eIF4E). Examples for cap-degrading enzymes are DcpS and Dcp1-2. Dcp2 is a bilobed enzyme composed of an N-terminal portion, which binds the activator Dcp1 and a catalytic domain, which carries out cap hydrolysis. Decapping Scavenger (DcpS) enzyme is an mRNA decapping pyrophosphatase.

It was suggested that targeting eIF4E is a potentially promising therapeutic strategy to inhibit aberrant pain plasticity. eIF4E integrates signals from two major signaling pathways, ERK and mTORC1, both of which have important functions in the development of pain. Further, eIF4E mainly regulates the translation of a subset of mRNAs involved in cell growth, proliferation, immune responses, and neuronal plasticity. Further, the 5'-cap is assumed to play a role in autoimmune diseases and metabolic diseases.

A further aspect thus relates to a use of a photocleavable 5'-cap analog as described herein for use as a medicament or for use in the diagnosis and/or treatment of disorders in connection with dysregulation in mRNA translation. Particularly, the photocleavable 5'-cap analog may be for use in the treatment of pain, cancer, autoimmune diseases or virus infection.

For the description of the 5'-cap analog and its elements reference is made to the description above. For use as a medicament or for use in treatment the 5'-cap analog preferably is a 5'-cap analog comprising (the naturally occurring) guanosine according to formula (I), for example a photocleavable 5'-cap analog according to formula (2). For diagnostic use or laboratory use the 5'-cap analog may be either a 5'-cap analog according to formula (I) or a 5'-cap analog comprising adenosine according to formula (II) such as according to formula (4).

Cleavage of the coumarin group from the N7-position of the guanosine-cap analog initially results in an unmethylated cap where, at least in mammalian cells, the native cap is quickly restored through cellular N7-methylation and thereupon translation occurs. This enables a temporal and spatial control of protein biosynthesis. The N7-modified 5'-cap analog therefore allows a pharmaceutical use under conditions that allow for remethylation.

The photocaged nucleotides are usable in the photo-activatable labelling of RNA or DNA. A further aspect thus relates to a photocleavable nucleoside or nucleotide analog as described herein for use in metabolic labelling. Metabolic labelling refers to methods in which the endogenous synthesis and modification machinery of living cells is used to incorporate detection tags into biomolecules. Typically, this is accomplished by culturing cells or organisms in media in which a specific natural molecular building block, e.g., nucleoside or nucleotide, has been replaced with a chemical analog. Cells use the analog instead of the natural biomolecule to synthesize or modify nucleic acids.

A further aspect relates to an RNA molecule comprising a photocleavable 5'-cap analog as described herein. For the description of the 5'-cap analog and its elements reference is made to the description above. The RNA may be an eukaryotic or a bacterial RNA. In embodiments, the RNA molecule comprises a photocleavable 5'-cap analog according to formula (I), for example a photocleavable 5'-cap analog according to formulas (VII) to (X) or formula (2). In other embodiments, the RNA molecule comprises a photocleavable 5'-cap analog according to formula (II), for example a photocleavable 5'-cap analog according to formulas (XI) to (XXII) or formula (4). In a preferred embodiment, the RNA molecule comprises a photocleavable 5'-cap analog according to formula (XIa).

RNA molecules are usable as therapeutic tools and an RNA molecule comprising the photocleavable 5'-cap analog is usable as a medicament. Another aspect relates to an RNA molecule comprising a photocleavable 5'-cap analog as described herein for use as a medicament or for use in the diagnosis and/or treatment of disorders in connection with dysregulation in mRNA translation. In preferred embodiments, the RNA molecule comprising a photocleavable 5'-cap analog as described herein is for use as a vaccine or for use in protein replacement therapy. Further, the RNA molecule comprising a photocleavable 5'-cap analog as described herein may be for use in the treatment of pain, cancer, autoimmune diseases or virus infection.

A further aspect relates to a diagnostic or pharmaceutical composition comprising a photocleavable 5'-cap analog as described herein and a pharmaceutically acceptable carrier. In embodiments, the diagnostic or pharmaceutical composition is usable in the diagnosis and/or treatment of disorders in connection with dysregulation in mRNA translation. Particularly, the composition comprising a photocleavable 5'-cap analog may be for use in the treatment of pain, cancer, autoimmune diseases or virus infection.

A further aspect relates to a diagnostic or pharmaceutical composition comprising an RNA molecule comprising a photocleavable 5'-cap analog as described herein and a pharmaceutically acceptable carrier. In embodiments, the diagnostic or pharmaceutical composition is usable in the diagnosis and/or treatment of disorders in connection with dysregulation in mRNA translation. In embodiments, the pharmaceutical composition is usable as a vaccine. In embodiments, the pharmaceutical composition is usable for protein replacement therapy. In further embodiments, the pharmaceutical composition may be usable in the treatment of pain, cancer, autoimmune diseases or virus infection.

A further aspect relates to a diagnostic composition comprising a photocleavable nucleoside or nucleotide analog as described herein and a pharmaceutically acceptable carrier.

The term "pharmaceutically acceptable" refers to molecular entities and compositions that do not produce an adverse, allergic or other untoward reaction when administered to a subject, such as, for example, a human, as appropriate. The composition may comprise a pharmaceutical carrier, which can be, for example, a solid, liquid, or gas. Suitable carriers preferably are liquid and correspond to the substances ordinarily employed in formulation technology for pharmaceutical formulations. For compositions convenient pharmaceutical media may be employed. For example, water, buffers, glycols, oils, alcohols and the like may be used to form liquid preparations such as solutions. The pharmaceutical composition may be produced under sterile conditions using standard pharmaceutical techniques well known to those skilled in the art.

A further aspect relates to a use of a photocleavable 5'-cap analog as described herein in a method of synthesizing an RNA molecule. For the description of the 5'-cap analog and its elements reference is made to the description above. In embodiments, the RNA molecule comprises a photocleavable 5'-cap analog according to formula (I), for example a photocleavable 5'-cap analog according to formulas (VII) to (X) or formula (2). In other embodiments, the RNA molecule comprises a photocleavable 5'-cap analog according to formula (II), for example a photocleavable 5'-cap analog according to formulas (XI) to (XXII) or formula (4). In a preferred embodiment, the RNA molecule comprises a photocleavable 5'-cap analog according to formula (XIa).

Using the photocleavable 5'-cap analog various RNA molecules, particularly messenger RNA molecules, which are protected from translation but upon irradiation are activatable, can be synthesized in an easy and straightforward way, such as, for example, by *in vitro* transcription (IVT). A further aspect relates to a use of a photocleavable 5'-cap analog as described herein for inhibiting cap-binding proteins or cap-degrading enzymes.

Apart from applications in mammals, also applications in or based on bacteria are of increasing interest, such as to study and activate the bacterial capped RNAs. For example, nucleoside tetraphosphate (Np₄N) capping of bacterial transcripts was found to be linked with cellular stress, and dinucleoside tetraphosphates have been deemed alarmones, i.e., second messengers that signal stress. Particularly caged dinucleoside tetraphosphates such as caged Ap₄A and Gp₄G and mixes may become usable as alarmones (caged alarmones) and studying and activating bacterial capped RNAs.

For example, tetraphosphate and pentaphosphate photocleavable 5'-caps having a PC-CouN7G modification are selected from PC-Cou⁷Gp₄A, m⁶Ap₄PC-Cou⁷G and m⁶Ap₅PC-Cou⁷G may be used for synthesizing an RNA molecule. Also the triphosphate caps of formulas (XVII) and (XVIII), the tetraphosphate caps of formulas (XIX) and (XX), and the pentaphosphate caps of formulas (XXI) and (XXII) are usable for synthesizing an RNA molecule. Further, photocleavable triphosphate 5'-caps having a PC-CouN1A modification selected from PC-Cou¹Ap₃A, PC-Cou¹Ap₃G, Gp₃PC-Cou¹A, m⁷Gp₃PC-Cou¹A, m⁶Ap₃PC-Cou¹A, and m⁶PC-Cou¹Ap₃A, or photocleavable tetraphosphate 5'-caps having a PC-CouN1A modification selected from PC-Cou¹Ap₄A, PC-Cou¹Ap₄m⁶A, Gp₄PC-Cou¹A, m⁷Gp₄PC-Cou¹A, m⁶PC-Cou¹Ap₄G, Cp₄PC-Cou¹A and Up₄PC-Cou¹A, or photocleavable pentaphosphate 5'-caps having a PC-CouN1A modification selected from PC-Cou¹Ap₅A, PC-Cou¹Ap₅m⁶A, m⁶Ap₅PC-Cou¹A, PC-Cou¹Ap₅m⁶A, PC-Cou¹Ap₅A, PC-Cou¹Ap₅m⁶A, PC-Cou¹Ap₅G and m⁶PC-Cou¹Ap₅G, are usable for synthesizing an RNA molecule.

Unless otherwise defined, the technical and scientific terms used herein have the same meaning as commonly understood by one of ordinary skill in the art to which this invention belongs.

The examples that follow serve to illustrate the invention in more detail but do not constitute a limitation thereof.

The figures show:
- Figure 1: An overview of the synthesis of a photocleavable 5'-cap analog according to an embodiment of the invention according to example 1.
- Figure 2: An overview of the synthesis of a photocleavable 5'-cap analog according to an embodiment of the invention according to example 2.
- Figure 3: shows the LC-MS Data (triple quad mass measurement) of the photocleavable 5'-cap analog of example 1 irradiated for 20 s with 450 nm.
- Figure 4: shows the LC-MS Data of the photocleavable 5'-cap analog of example 2 irradiated for 1 min with 450 nm.
- Figure 5: shows the results of an PAA-gel electrophoresis of Cypridina Luciferase mRNA capped with the photocleavable 5'-cap analog of example 1 (denoted DEACM) compared to commercial non-modified cap analogs.
- Figure 6: shows translation in cells as luminescence measurements after irradiation of GLuc-mRNA capped with the photocleavable 5'-cap analog of example 1 (denoted N7Cou) and a commercial non-modified cap analog (GpppG).
- Figure 7: shows confocal laser scanning microscopy (CLSM) images of HeLa cells transfected with eGFP mRNA capped with either the photocleavable 5'-cap analog of example 1 (denoted DEACM -N7-GpppG) or commercial non-modified cap analogs (m7GpppG, GpppG, ApppG) kept in the dark or after irradiation.

### Materials:

All chemicals were purchased from Alfa Aesar, Applichem, Fluorochem, Sigma Aldrich or TCI unless otherwise noted. The HPLC grade acetonitrile was purchased from VWR. Anhydrous zinc chloride (ZnCl₂) was dried by heating at 220 °C under high vacuum for ~3.5 h and cooled to room temperature under argon. All other components were used without further purification. Triethylamine (N(Et)₃) was freshly dried over CaH₂ overnight at room temperature. Unless stated otherwise, solvents were evaporated at 40 °C.

### Methods

### ¹H, ¹³C and ³¹P NMR spectrometry:

¹H, ¹³C and ³¹P NMR spectra were measured on Agilent DD2 600 spectrometer (600 MHz, 151 MHz and 243 MHz) and Agilent DD2 500 spectrometer (500 MHz, 126 MHz and 202 MHz). The measurements were performed in DMSO-*d6* or D₂O and referred to residual solvent signal. Complete assignment (if present) is based on heteronuclear correlation experiments HSQC and H, C-HMBC. Chemical shifts (*δ*) are in ppm and coupling constants (*J*) in Hz. The numbering system for the assignment of NMR signals is given for the majority of the compounds individually.

### Mass spectrometry:

High resolution mass spectra were measured on Orbitrap LTQ XL (Thermo Fisher Scientific) spectrometer using ESI technique and Orbitrap Velos Pro (Thermo Fisher Scientific) spectrometer using ESI technique.

### High performance liquid chromatography (HPLC):

HPLC analysis was performed on an Agilent 1260 Infinity HPLC equipped with a diode array detector (DAD) (190-640 nm) using a Nucleodur^{®} C18 Pyramid reversed-phase column (5 µm, 125 × 4 mm) from Macherey-Nagel. Elution was performed at a flow rate of 1 mL/min applying a linear gradient for buffer A (100 mM K-Phosphate, pH = 6.5) and buffer B (1: 1 buffer A : acetonitrile).

### Flash chromatography:

For flash chromatography, Interchim puriFlash^{®} XS520Plus with multi λ absorbance detector and various types of columns were used: a) 330 g column, 50 µm spherical silica gel, Interchim [PF-50SIHP-F0330]; b) 120 g column, 50 µm spherical silica gel, Interchim [PF-50SIHP-F0120]; c) 40 g column, 25 µm spherical silica gel, Interchim [PF-25SIHC-F0040].

For reversed-phase (RP) flash chromatography and preparative HPLC, Büchi PrepChrom C-700 multi λ absorbance detector and various types of columns were used: a) Teledyne ISCO columns RediSepRf^{®} variation HP C18 Aq GOLD in sizes 50 g, 100 g and 150 g (RP flash chromatography); b) Macherey-Nagel VP 250/21 NUCLEODUR C18 Pyramid, 10 µm [REF 762285.210] (preparative HPLC).

### Example 1

Synthesis of 7-((7-(diethylamino)-2-oxo-2H-chromen-4-yl)methyl)-guanosine phosphate according to formula (1) and 7-((7-(diethylamino)-2-oxo-2H-chromen-4-yl)methyl)-guanosin-5'-yl)-P3-guanosin-5'-yl triphosphate according to formula (2)

### 1.1 Synthesis of 4-(bromomethyl)-7-(diethylamino)-2H-chromen-2-one (DEACM-Br)

DEACM-OH was synthesized following the literature procedures. (Chem. Sci., 2019,10, 4209-4219; Chem.Commn., 2018, 54, 10371-10374). DEACM-OH (5 g, 20 mmol, 1 eq) was vacuum dried in 250 mL round bottom flask, and cooled to ice bath temperatures. 120 mL of dry DCM (0.16 M) was added, followed by dry triethylamine (5.6 mL, 40 mmol, 2 eq). After 10 min, methanesulfonyl chloride (4.7 mL, 60 mmol, 3 eq) was added dropwise in 3-5 min. After 45 min, the reaction was diluted with another 30 mL of DCM, and washed the organic layer with sat. NaHCO₃ (35 mL × 3). The organic layer was separated, dried over sodium sulphate (no brine wash) and concentrated under reduced pressure. Without further purification, proceeded to next step. The resulting crude mesylate derivative (8.5 g, 26 mmol, 1 eq) was vacuum dried in 500 mL round bottom flask, then added lithium bromide (9.0 g, 104 mmol, 4 eq) followed by DCM (80 mL, 0.33 M). The reaction was stirred at room temperature. After 8 h, the reaction was washed with ddH₂O (30 mL × 4) and dried over sodium sulfate, filtered and concentrated. The resulting crude was purified on puriflash using 50SIHP-F0320 column using cyclohexane and ethyl acetate (10-60 % of ethyl acetate).

### 1.2 Synthesis of 7-((7-(diethylamino)-2-oxo-2H-chromen-4-yl)methyl)-guanosine phosphate according to formula (1)

In a 10 mL Schlenk tube, guanosine monophosphate disodium, GMP (0.17 g, 0.41 mmol, 1 eq) and DEACM-Br (0.42 g, 1.37 mmol, 3 eq) were vacuum dried and dissolved in dry DMSO (5 mL). The reaction was heated to 40 °C. Initially the reaction mixture was turbid, but upon heating became a clear solution. After stirred for 21 h, the reaction was diluted with another 1 mL of DMSO. The resulting residue was purified by reversed-phase (RP) flash chromatography (aq-C18 column 150 g; pre-equilibrated with 0.1 M TEAB, eluents TEAB (0.1 M)/CH₃CN, gradient 0-50 % of CH₃CN, over 15 min with a flow rate of 65 mL/min). The fractions containing the product were combined and lyophilized. The obtained compound was in the form triethylammonium salt.

7-((7-(diethylamino)-2-oxo-2H-chromen-4-yl)methyl)-guanosine phosphate (1) according to formula (1) was confirmed via ¹H NMR, ³¹P NMR, and ESI-HRMS.

### 1.3 Synthesis of 7-((7-(diethylamino)-2-oxo-2H-chromen-4-yl)methyl)-guanosin-5'-yl)-P3-guanosin-5'-yl triphosphate according to formula (2)

In a 10 mL Schlenk tube, anhy. zinc chloride (0.6 g, 4.3 mmol, 40 eq) was weighed and heated at 200 °C under vacuum for ~2 h. After reaching room temperature, triethylammonium salt of 7-((7-(diethylamino)-2-oxo-2H-chromen-4-yl)methyl)-guanosine phosphate as received from Example 1.2 (65 mg, 0.1 mmol, 1 eq), followed by dry DMF (7 mL) were added. After complete dissolution, GDP imidazolate (176 mg, 0.32 mmol, 3 eq) was added and shaked until clear solution. The resulting solution was vigorously stirred for 21 h. The reaction was quenched by addition of ddH2O (1 mL) and an EDTA solution (2.25 mL, 0.5 M). After 15 min of vigorous stirring, the resulting residue was purified by reversed-phase (RP) flash chromatography (aq-C18 column 150 gm; pre-equilibrated with 0.1 M TEAB, eluents TEAB (0.1 M)/CH₃CN, gradient 0-50 % of CH₃CN over 15 min with a flow rate of 65 mL/min). The purified product was dissolved in 1.2 mL of water and injected to POROS HQ-50 column (pre-equilibrated with ddH₂O, eluents ddH₂O/TEAB (1 M), gradient 0-100 % of TEAB over 30 min). The fractions containing the desired product were combined and lyophilized.

The obtained compound was in the form triethylammonium salt. 7-((7-(diethylamino)-2-oxo-2H-chromen-4-yl)methyl)-guanosin-5'-yl)-P3-guanosin-5'-yl triphosphate according to formula (2) was confirmed via ¹H NMR, ³¹P NMR, and ESI-HRMS. ESI-HRMS: Calculated for [M-H]⁻² C₃₄H₄₀N₁₁O₂₀P₃ 507.5837, found 507.5825. For *in vitro* transcription, the triethyl ammonium ions are exchanged to sodium ions using Dowex^{®} 50WX8 (H) 100-200 mesh (Across Organics) ion-exchange resin.

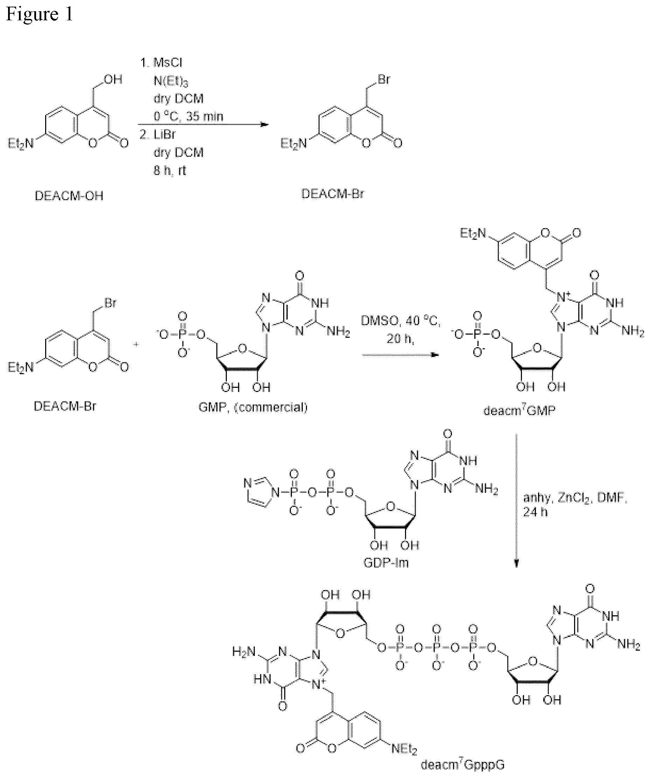

An overview of the synthesis of the photocleavable 5'-cap analog with photocleavable coumarin group in N7-position is shown in Figure 1.

### Example 2

Synthesis of N1-(7-(Diethylamino)-coumarin-4-yl)methyl adenosine-5'-monophosphate according to formula (3) and P1-guanosine-5'-yl)-P3-(N1-(7-(diethylamino)-coumarin-4-yl)methyl-adenosine-5'-yl triphosphate according to formula (4)

### 2.1 Synthesis of N1-(7-(Diethylamino)-coumarin-4-yl)methyl adenosine

In a 50 mL Schlenk flask adenosine (0.43 g, 1.62 mmol, 1.0 eq) was subjected to vacuum-argon cycles for 3-5 min and placed in preheated oil bath at 40 °C. Then dry DMSO (20 mL, 0.08 M) was added, followed by DEACM-Br (1.5 g, 4.85 mmol, 3.0 eq). The reaction was stirred for 26 h at 40 °C. The resulting reaction crude was directly purified by reversed-phase (RP) flash chromatography (aq-C18 column 150 gm; eluents ddH₂O/CH₃CN, gradient 0-55 % of CH₃CN, over 15 min with a flow rate of 65 mL/min). The fractions containing the product were combined and lyophilized.

### 2.2 Synthesis of N1-(7-(Diethylamino)-coumarin-4-yl)methyl adenosine-5'-monophosphate according to formula (3)

In a 50 mL Schlenk tube, N1-(7-(Diethylamino)-coumarin-4-yl)methyl adenosine (0.2 g, 0.4 mmol, 1 eq) was kept at ice bath temperature under argon flow. To this anhy. trimethyl phosphate (4 mL), followed by freshly distilled phosphorus oxychloride (0.06 mL, 0.64 mmol, 1.6 eq) was added dropwise. After vigorous stirring at the same ice bath temperature for 2 h, the reaction was quenched with TEAB (1 M, 12 mL). The pH of aqueous medium was found to be between pH 8-9. This reaction mixture was washed with MTBA (30 mL * 5) and once with EtOAc (25 mL). The aqueous layer was degassed by argon bubbling and concentrated to 3-6 mL. The resulting residue was purified by reversed-phase (RP) flash chromatography (aq-C18 column 150 gm; pre-equilibrated with 0.1 M TEAB, eluents TEAB (0.1 M)/CH₃CN, gradient 0-50 % of CH₃CN, over 15 min with a flow rate of 65 mL/min). The fractions containing the product were combined and lyophilized. The desired compound was obtained in the form of triethylammonium salt and appeared as yellow solid.

N1-(7-(Diethylamino)-coumarin-4-yl)methyl adenosine-5'-monophosphate according to formula (3) was confirmed via ¹H NMR, ¹³C NMR, and ³¹P NMR.

### 2.3 Synthesis of P1-guanosine-5'-yl)-P3-(N1-(7-(diethylamino)-coumarin-4-yl)methyl-adenosine-5'-yl triphosphate according to formula (4)

In a 10 mL Schlenk tube, anhy. zinc chloride (0.94 g, 6.47 mmol, 40 eq) was weighed and heated at 200 °C under vacuum for ~2 h. After reaching room temperature, triethylammonium salt of N1-(7-(Diethylamino)-coumarin-4-yl)methyl adenosine-5'-monophosphate (0.1 g, 0.17 mmol, 1 eq), followed by dry DMSO (8 mL) were added. After complete dissolution, GDP-imidazolide (0.28 g, 0.52 mmol, 3 eq) was added and shaked until clear solution. The resulting solution is vigorously stirred for 36 h. The reaction was quenched by addition of ddH2O (1 mL) and an EDTA solution (2 mL, 0.5 M). After 15 min of vigorous stirring, the resulting residue was purified by reversed-phase (RP) flash chromatography (aq-C18 column 150 gm; pre-equilibrated with 0.1 M TEAB, eluents TEAB (0.1 M)/CH₃CN, gradient 0-50 % of CH₃CN over 15 min with a flow rate of 65 mL/min). The purified product was dissolved in 1.2 mL of water and injected to POROS HQ-50 column (pre-equilibrated with ddH₂O, eluents ddH₂O/TEAB (1 M), gradient 0-100 % of TEAB over 30 min). The fractions containing the desired product were combined and lyophilized. in the form of triethylammonium salt

The obtained compound was in the form triethylammonium salt. P1-guanosine-5'-yl)-P3-(N1-(7-(diethylamino)-coumarin-4-yl)methyl-adenosine-5'-yl triphosphate according to formula (4) was confirmed via ¹H NMR, ¹³C NMR, ³¹P NMR, and ESI-HRMS. ESI-HRMS: calculated for [M-H]²⁻, C₃₄H₄₀N₁₁O₁₉P₃⁻² 499.5862, found 499.5861.

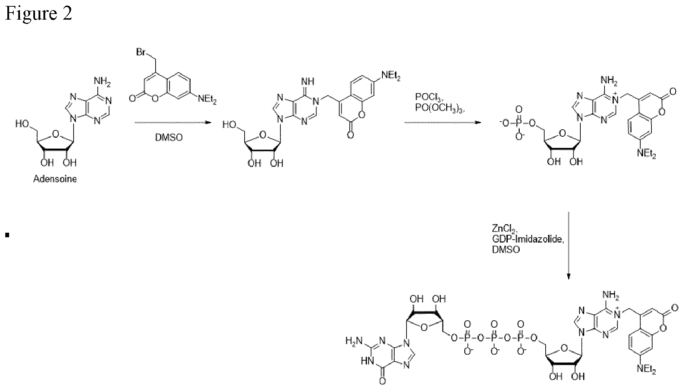

An overview of the synthesis of the photocleavable 5'-cap analog with photocleavable coumarin group in N7-position of Example 2 is shown in Figure 2.

### Biological and biochemical assays:

Guanosine 5'-Monophosphate Disodium Salt Hydrate was purchased from TCI. 7-Diethylamino-4-methylcoumarin was purchased from Alfa Aesar.

**Table 9: Enzymes and inhibitors:**

| Enzyme | Manufacturer |
|---|---|
| DNase I [1 U/µL] | Thermo Fisher Scientific |
| Phusion^{®} DNA Polymerase [2 U/µL] | Thermo Fisher Scientific |
| Pyrophosphatase [0.1 U/µL] | Thermo Fisher Scientific |
| RiboLock RNase Inhibitor [20 U/µL] | Thermo Fisher Scientific |
| T7-RNA-Polymerase [20 U/µL] | Thermo Fisher Scientific |
| XRN1 [1 U/µL] | New England Biolabs GmbH |
| 5'-Polyphosphatase [20 U/µL] | Biozym Scientific GmbH |
| PaqCI | New England Biolabs GmbH |

**Table 10: Cap analogs and NTPs:**

| Name | Manufacturer |
|---|---|
| ApppG | Jena Bioscience GmbH |
| GpppG | Jena Bioscience GmbH |
| m⁷GpppG | Jena Bioscience GmbH |
| NTP mix | Jena Bioscience GmbH |
| m⁵C | Jena Bioscience GmbH |
| m1Ψ | Jena Bioscience GmbH |

**Table 11: Buffers and solvents:**

| Name | Composition |
|---|---|
| APS stock solution | 10 % (w/v) APS in ddH₂O |
| 6x DNA-Loading Dye | 10 mM Tris; 60 % (v/v) Glycerol; 60 mM EDTA; 0.03 % (w/v) bromphenolblue and xylencyanol FF; pH 7.6 |
| HF-buffer (5x) | Composition classified |
| RNA-Loading Dye (2x) | 95 % (v/v) Formamide, 87 µM SDS, 0.5 mM EDTA |
| TAE-buffer (50x) | 2 M Tris; 50 mM EDTA; pH 8.5 |
| TBE-buffer (5x) | 0.45 M Tris; 0.45 M boric acid; 0.01 M EDTA |
| Transcription buffer (5x) | 200 mM Tris; pH 7.9; 30 mM MgCl₂; 50 mM DTT; 50 mMNaCl; 10 mM spermidine |
| MEM | 1 % glutamine; 1 % 100x non-essential amino acids; 1x fetal calf serum |
| PBS (10x) | 1.4 M NaCl; 0.027 M KCl; 0.1 MNa₂HPO₄; 0.02 M KH₂PO₄ |
| LB-medium | 10 g tryptone, 5 g yeast extract, 10 g NaCl, 900 mL ddH₂O |

### mRNA synthesis (in vitro transcription)

The eGFP and *Gaussia* Luciferase (GLuc) mRNAs were synthesized using the runoff template of the pRNA2-(A)128 vector. The pRNA2-(A)128 vector was a gift from Stephen Ikeda (Addgene plasmid #174006; http://n2t.net/addgene: 174006; RRID: Addgene 174006). For the preparation of the runoff template, the plasmid DNA (pDNA, 7 µg) was linearized using restriction enzyme PaqCI (NEB, 10 U) and PaqCI activator (NEB, 1 U) in 1 × CutSmart^{™} buffer according to the manufacturer's instructions (NEB). After incubation at 37 °C for 1 h, the restriction enzyme was inactivated at 65 °C for 10 min. To prevent religation, dephosphorylation of the linearized DNA was performed in the presence of Fast AP (6 U) for 15 min at 37 °C, followed by inactivation at 75 °C for 5 min. The runoff plasmid was purified using the NucleoSpin Gel and PCR Clean-up kit (Macherey-Nagel). The dsDNA concentration was measured via absorption at 260 nm with a Tecan Infinite M1000 PRO instrument. And the purity of the DNA template was confirmed by agarose gel electrophoresis.

For the *in vitro* T7 transcription, the resulting runoff template (400 ng) was transcribed using A/m⁵C/m1ΨTP mix (Jena Bioscience, 0.5 mM), GTP (0.1 mM), m⁷GpppG, ApppG, GpppG or DEACM⁷GpppG cap analog according to formula (2) (1 mM), RiboLock RNase Inhibitor (30 U), T7 RNA polymerase (50 U) and pyrophosphatase (0.1 U) for 6 h at 37 °C. Remaining DNA template was digested by incubation with DNase I for 1 h at 37 °C. The RNA was purified using the RNA Clean & Concentrator^{™}-5 Kit (*Zymo Research*). Uncapped RNA (5 µg) was removed by incubation with 5'-Polyphosphatase (Biozym, 10 U) at 37 °C for 1 h in 1 × 5'-polyphosphatase buffer, followed by the addition of MgCl₂ (10 mM) and XRN-1 (New England Biolabs, 1 U). The RNA was again purified, stored at -20 °C and the quality was assessed via 7.5 % denaturing PAGE. The gel was imaged using the Typhoon FLA 9500 scanner.

### HeLa cell culture and irradiation for microscopy

HeLa cells (Sigma Aldrich) were cultured in growth media comprising MEM Earle's media (Merck) supplemented with fetal calf serum (10 %) L-glutamine (2 mM), non-essential amino acids (1 %), penicillin and streptomycin (1 %) at 37 °C in 5 % CO₂. One day before transfection, cells were seeded (1 mL) on 15 mm cover slips in a 12-well cell culture plate (2 × 10⁵ cells per well). Transfection of eGFP mRNA (1 µg) was performed using Lipofectamine Messenger Max (Invitrogen, 3 µL) in Opti MEM (100 µL). Cells were incubated for 6 h at 37 °C in a total volume of 1 mL, followed by media exchange to fresh growth media. Cells were kept in the dark, irradiated at 450 nm for 10 s or irradiated at 450 nm for 20 s. Then, media was replaced with fresh growth media and cells were incubated for another 18 h at 37°C.

### Microscopy of HeLa cells

24 h post-transfection, cells were washed twice with 1 × PBS, fixed in 4 % paraformaldehyde (500 µL per well, 10 min incubation at RT) and washed again twice with 1 × PBS. Nuclei were stained with DAPI (10 µg/µL). After washing twice with 1 × PBS and once quickly with ddH₂O, the coverslips were quickly inverted and mounted on microscope glass slides using Aqua-Poly/Mount. Fixed cells were imaged on a Leica A TCS SP8 confocal laser scanning microscope with a 10x water-immersion objective. Images were taken at a green channel for eGFP fluorescence (λₘₐₓ = 488 nm, λₑₘ = 510 nm, excitation laser = 473 nm, scanning filter LPB).

### Light sources

Blue LED (≥20 µW/mm², *λₘₐₓ* = 450 nm) was used for the irradiation of cells. The LED was obtained from Thorlabs and installed in a self-made LED-box. The temperature of the box was constantly at 23 °C.

### Gaussia luciferase (GLuc) Luminescence measurement

For luminescence measurements, the supernatant of HeLa cells transfected with GLuc-mRNA and incubated for 24 h was utilized. With 1xPBS, the supernatant was diluted 1: 10. Using a luciferase experiment using Beetle-juice Buffer and coelenterazine, the translation efficiency of reporter mRNAs was measured (pjk GmbH). The activity of the Luciferase enzyme was measured by adding 50 µL of a freshly prepared substrate solution to the translation mixture. Using a Tecan infinite^{®} m1000pro microplate reader with a 3 s (duration of signal acquisition) acquisition period, luminescence was measured.

### Translation and irradiation in HeLa cells for Luminescence assay

20 h before transfection, HeLa cells were placed into a 96-well plate. 3 × 10⁴ cells per well were grown in growth Media (MEM, 10% FCS, 1% glutamine, 1% Penicillin, 1% Streptomycin, and 1% non-essential amino acids). For transfection, the growth medium was replaced with MEM-media containing 0.3 µL of Metafectene^{®} Pro (0.6 µL) and 100 ng of GLuc-mRNA per well. 6 h after transfection, the medium was replaced with MEM-media, followed by 450 nm irradiation and another MEM-media exchange. After 24 h, the supernatant was utilized for further luminescence assays. The ApppG-capped mRNA values reflect translation independent of the cap and were thus subtracted from the other values.

### Denaturing urea-polyacrylamide gel electrophoresis (dPAGE)

Long RNAs were analyzed using denaturated PAGE. Table 8 describes the composition of a 7.5% gel. In the last stage, TEMED and APS were added to induce polymerization. Prior to loading the samples, the polymerized polyacrylamide gels were pre-run for 10 minutes at 15 W. By rinsing the pockets with 1x TBE buffer, residual urea may be eliminated. With a 2x RNA loading dye, samples and a marker were combined. RiboRuler LR (Thermo Fisher Scientific) was utilized as a size indicator. After loading the samples, separation was done at 12 W for 1 hour in 1x TBE buffer. RNA was visualized by SYBR^{™}-Gold staining (dilution of stock solution 1:20000) for 10 min, followed by imaging with a Typhoon FLA9500 scanner after Cy5 channel scanning (excitation SYBR Gold 473 nm).

**Table 12: components for a dPAGE gel**

| Component | Volume |
|---|---|
| Rotiphorese^{®} Sequencing gel concentrate | 3 mL |
| Urea (8 M urea in ddH₂O) | 5 mL |
| 5 × TBE buffer | 2 mL |
| TEMED | 5 µL |
| APS (10 %) | 50 µL |

### LC-QqQ_MS analysis:

The HPLC - triple-quadrupole mass spectrometry equipment was utilized to determine mass spectra. Agilent 1260 Infinity II with dual absorbance detector (G7114A) HPLC and Agilent Ultivo mass spectrometer with JetStream ion source constitute the system (type of ESI). The column utilized was an Agilent Poroshell 120 EC-C18 (3.0×150mm/2.7µm) column. The polar compounds were analyzed using the LC technique (eluent 20 mM NH₄OAc buffer (pH = 6)/CH₃CN, gradient 0-30%, column temperature =20°C).

### Example 3

### Determination of the effect of irradiation on the photocleavable 5'-cap analogs of formula (2)

Samples of the photocleavable 5'-cap analog of formula (2) (here denoted deacm⁷GpppG) were irradiated for 20 s with 450 nm.

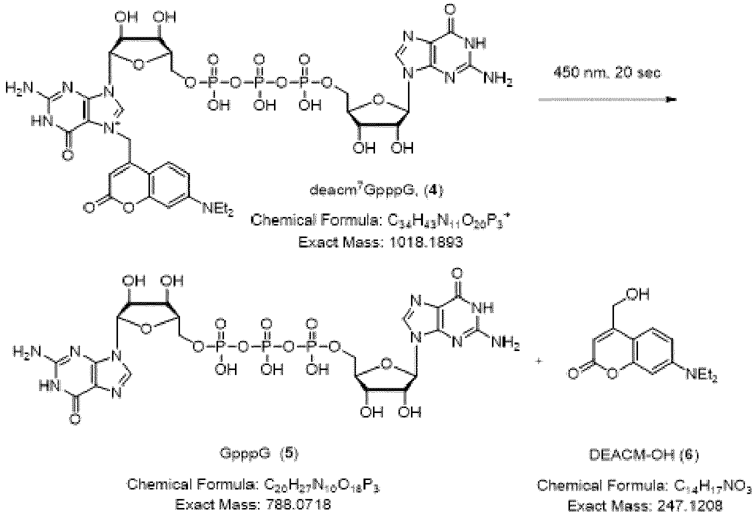

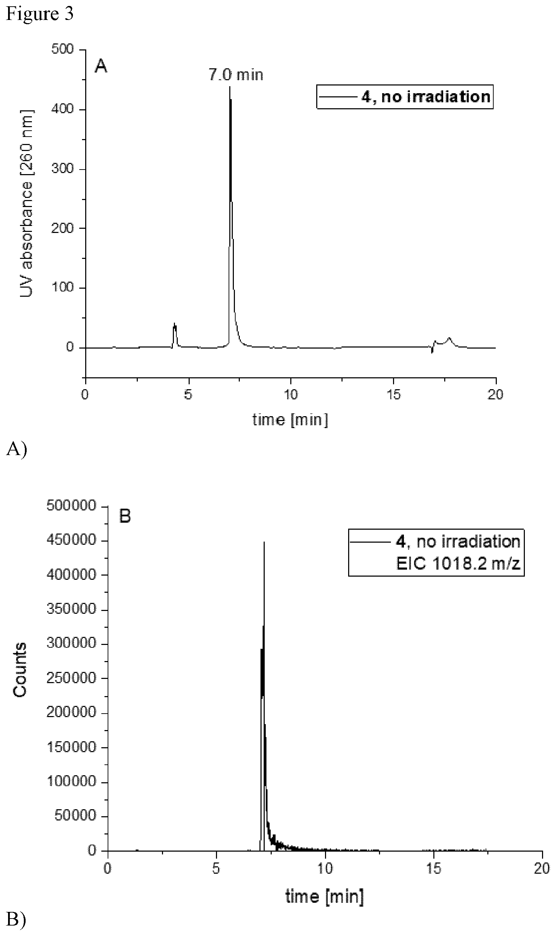

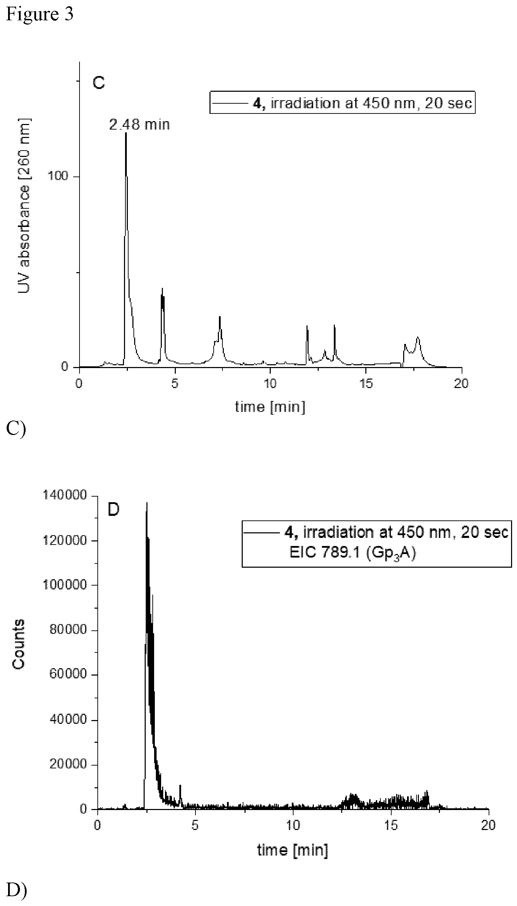

The Figure 3 shows the results of the cap (denoted "N7-DEACM" in Figure 3) with LCMS. Figures 3A and 3C represents UV absorbance at 260 nm of the N7-DEACM cap 4 and its photolysis products after irradiation at 450 nm, respectively. Figures 3B and 3D show extracted ion count (EIC) of the N7-DEACM cap 4 and its photolysis product after irradiation at 450 nm, respectively. The Extracted Ion Count (EIC) of 789.1 m/z represents the mass of the uncaged GpppG cap analog (calculated mass: [C₂₀H₂₇N₁₀O₁₈P₃]⁺ = 788.0718 [M]⁺; found: 789.1 [M+H]⁺).

This shows that irradiation for 20 s with 450 nm resulted in successful and efficient photocleavage of the 5'-cap analog of formula (2).

### Example 4

### Determination of the effect of irradiation on the photocleavable 5'-cap analogs of formula (4)

Samples of the photocleavable 5'-cap analog of formula (4) (below denoted Gp₃deacm¹A) were irradiated for 1 min with 450 nm.

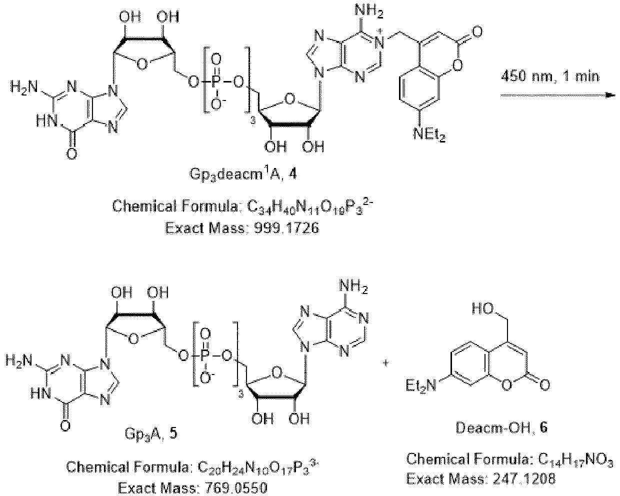

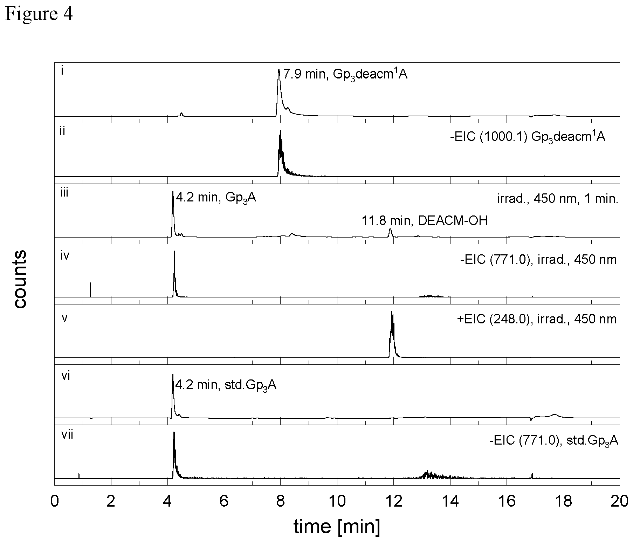

The Figure 4 shows the results of the LCMS analysis of 5'-cap analog of formula (4) (Gp₃deacm¹A) and photocleaved products, where i, iii and vi represents UV absorbance at 260 nm of the Gp3deacm¹A cap, its photolysis products after irradiation at 450 nm and Gp₃A standard for reference respectively. ii, iv, v and vii are extracted ion count (EIC) of the Gp3deacm¹A cap, the photolysis product Gp₃A, photolysis by-product coumarin alcohol, and Gp₃A standard for reference respectively.

This shows that irradiation with 450 nm resulted in successful and efficient photocleavage of the 5'-cap analog of formula (4).

### Example 5

### Synthesis of RNA using the photocleavable 5'-cap analog of formula (2)

The photocleavable 5'-cap analogs of formula (2) was used for *in vitro* transcription of Cypridina Luciferase mRNA as described above and analyzed via agarose gel electrophoresis.

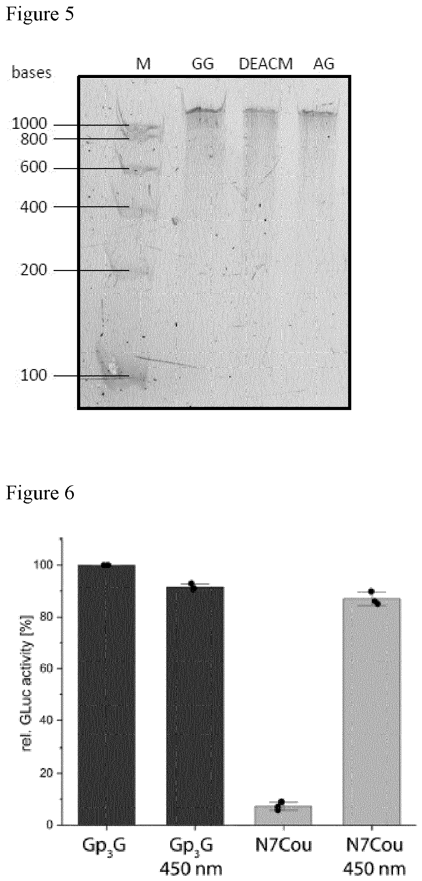

The Figure 5 shows the gel analysis of Cypridina Luciferase mRNA capped with the photocleavable 5'-cap analog of formula (2) comprising a N7-coumarin group, denoted "DEACM" in Figure 5, and mRNA capped with commercial non-modified cap analogs GpppG (denoted "GG") and ApppG (denoted "AG") (both Jena Bioscience) using a 7.5 % TBE mini gel. "M" denotes the Riboruler low range RNA ladder. As can be taken from the figure 5, the photocleavable 5'-cap analog of formula (2) was incorporated into the RNA as were the commercial non-modified cap analogs used as controls.

### Example 6

### Determination of in vitro translation using the photocleavable 5'-cap analog of formula (2)

The photocleavable 5'-cap analog of formula (2) was used for *in vitro* transcription to make GLuc mRNA as described above. This was used for *in vitro* translation and Luminescence measurement as described above. The commercial non-modified cap analog GpppG (denoted "Gp₃G") was used as positive control. Samples were irradiated at 450 nm for 20 s. The experiment was performed in quadruplicates. The GLuc-mRNA capped with the photocleavable 5'-cap analog of formula (2) is denoted "N7Cou".

Figure 6 shows the results of the *in vitro* translation assay and luminescence measurement with DEACM-N7-GpppG mRNA and GpppG-capped GLuc-mRNA. The values were normalized to the non-irradiated sample with GpppG capped mRNA (100%).

As can be taken from Figure 6, GLuc protein expression levels of DEACM GpppG mRNA (N7Cou) were strongly reduced in HeLa cells. However, after irradiation of the cells with 450 nm for 20 sec, the expression levels were comparable to native mRNA.

### Example 7

### Determination of in-cell translation upon irradiation of RNA modified with the photocleavable 5'-cap analog of formula (2)

In-cell translation and irradiation in cell culture of mammalian HeLa cells was performed as described above. GLuc mRNA was capped with the photocleavable 5'-cap analog of formula (2) (denoted DEACM⁷GpppG). Further GLuc mRNA was capped with the non-methylated cap analog denoted "GpppG" (Jena Bioscience). The commercial non-modified cap analog denoted "m⁷GpppG" (Jena Bioscience) was used as a positive control. ApppG capped mRNA, which is not recognized by the translation initiation factor, served as negative control. HeLa cells were transfected with the differently capped mRNAs, i.e. DEACM⁷GpppG-capped mRNA, GpppG-capped mRNA, m⁷GpppG-capped mRNA or ApppG-capped mRNA. Cells were kept in the dark or irradiated either at 450 nm for 10 s or for 20 s. This experiment was performed as independent triplicates.

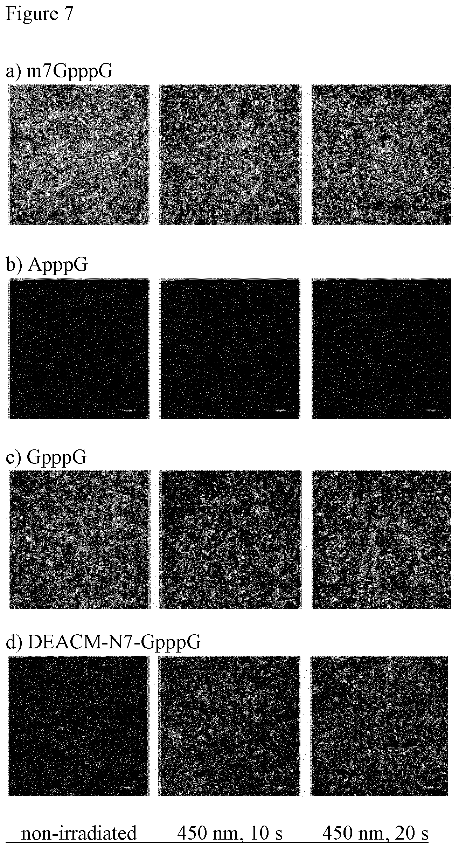

The Figure 7 shows the results of the confocal laser scanning microscopy (CLSM) images of fixed HeLa cells transfected with differently capped eGFP mRNA, where Figure 7a) shows m7GpppG, Figure 7b) GpppG, Figure 7c) ApppG, and Figure 7d) DEACM⁷GpppG. It can be seen that cellular methylation of the N7-position of the non-methylated cap analog GpppG widely restored translation in cells both kept in the dark or irradiated. As can be taken from Figure 7, in cells transfected with the photocleavable 5'-cap analog of formula (2) ("DEACM-N7-GpppG") translation was severely reduced when kept in the dark, while restored to a level comparable with cells transfected non-methylated cap analog GpppG.

This shows that a photocleavable 5'-cap analog is provided that allows a photocontrollable regeneration of the non-methylated cap upon radiation, which allows to restore the native cap upon cellular N7 methylation.

The work leading to this invention has received funding from the European Research Council (ERC) under EU Horizon 2020 research and innovation programme under grant agreement No. 772280.

## Claims

1. A photocleavable 5'-cap analog according to formulas (I) or (II) as given as follows or salts thereof, wherein the 5'-cap analog comprises a photocleavable group PC-Cou at the N7-position of guanosine or the N1-position of adenosine: 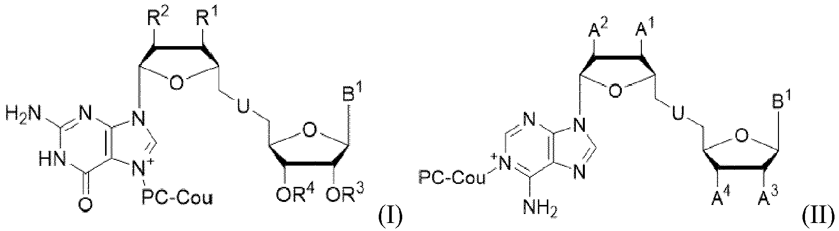 wherein:
R¹, R² are independently H, OH or OCH₃;
R³ is H or CH₃;
R⁴ is H or an element according to formula (III) 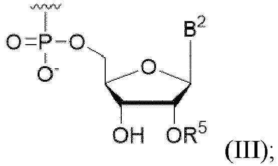 wherein R⁵ is H or CH₃;
U is a modified or unmodified oligophosphate or methyleneoligophosphate group;
A¹ is R¹ or OR⁴;
A² is R² or OR³;
A³ is OR³ or R²;
A⁴ is OR⁴ or R¹;
B¹, B² is a modified or unmodified nucleobase; and
PC-Cou is a photocleavable coumarin group.

2. A photocleavable nucleoside or nucleotide analog or salt thereof, wherein the analog is a nucleoside or nucleotide analog according to formulas (IV), (V) or (VI) as given as follows and comprises a photocleavable group PC-Cou at the N7-position of guanosine, at the N1-position of adenosine, or at the N3-position of cytidine: 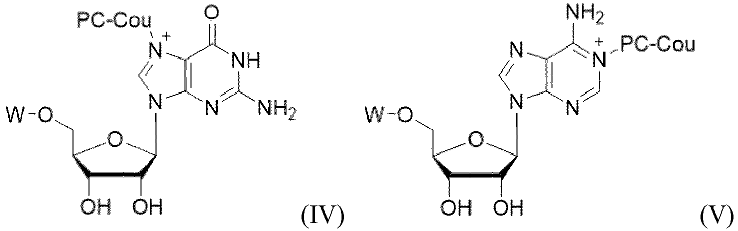 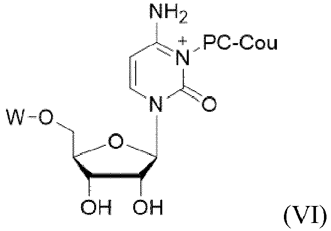 wherein:
W is H or a modified or unmodified mono-phosphate, di-phosphate, or tri-phosphate group, and
PC-Cou is a photocleavable coumarin group.

3. The photocleavable 5'-cap analog or the photocleavable nucleoside or nucleotide analog according to claims 1 or 2, wherein PC-Cou is a photocleavable coumarin selected from the group of (coumarin-4-yl)methyl compounds according to formulas (P1) to (P23) as given as follows: 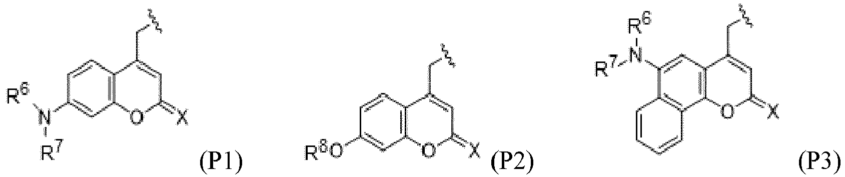 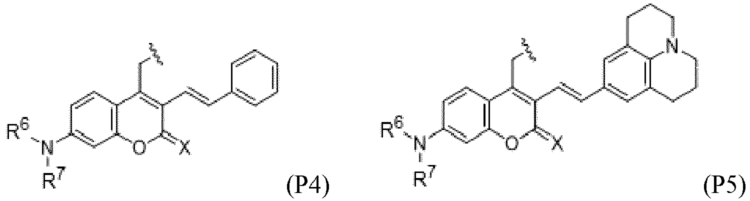 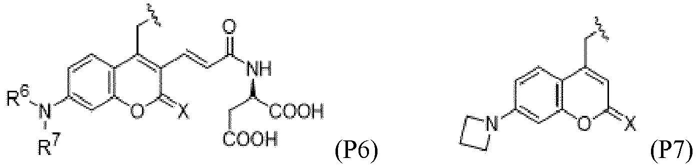 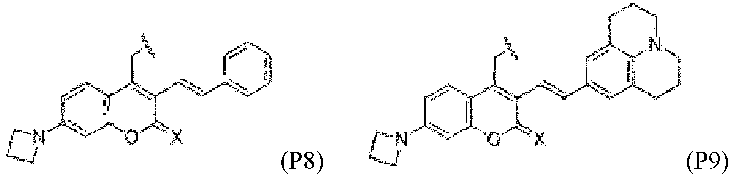 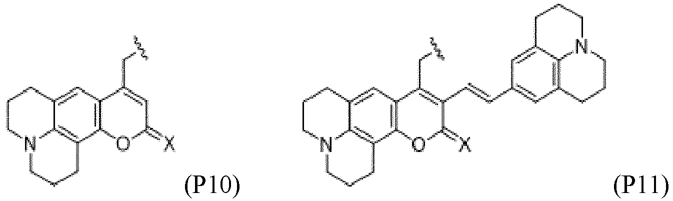 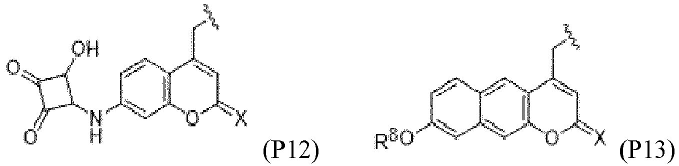 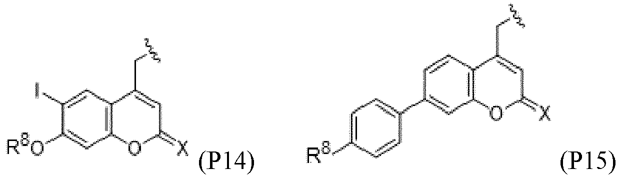 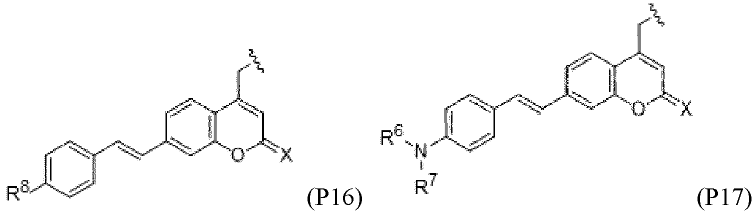 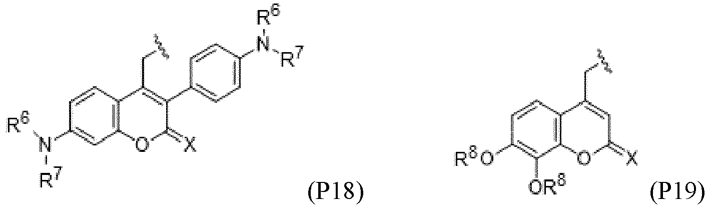 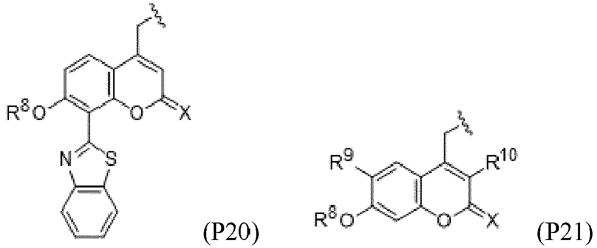 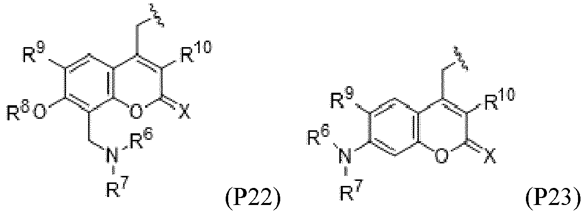 wherein:
R⁶, R⁷ are independently selected from the group of H, C₁-C₁₀ alkyl, C₃-C₇ cycloalkyl, and 2-propynyl;
R⁸ is selected from the group of H, C₁-C₁₀ alkyl, C₁-C₁₀ COOH, and C₁-C₁₀ alkoxy;
R⁹, R¹⁰ are independently selected from the group of H, I or Br; and
X is selected from the group of O, S and Se.

4. The photocleavable 5'-cap analog or the photocleavable nucleoside or nucleotide analog according to any one of the foregoing claims, wherein PC-Cou is the photocleavable [7-(diethylamino)coumarin-4-yl]methyl according to formula (P24) as given as follows: 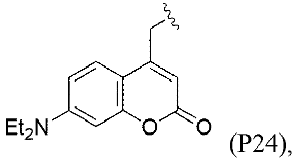 wherein Et is ethyl.

5. The photocleavable 5'-cap analog according to any one of claims 1, 3, or 4, wherein B¹ and B² independently are a natural nucleobase selected from guanine or adenine.

6. The photocleavable 5'-cap analog according to any one of claims 1, or 3-5, wherein the photocleavable 5'-cap analog has a structure according to formula (2) as given as follows or salts thereof, wherein Et is ethyl:

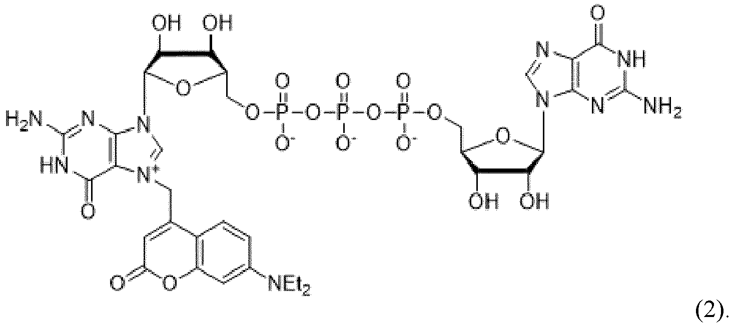

7. The photocleavable 5'-cap analog according to any one of claims 1, or 3-5, wherein the photocleavable 5'-cap analog has a structure according to formula (4) as given as follows or salts thereof, wherein Et is ethyl:

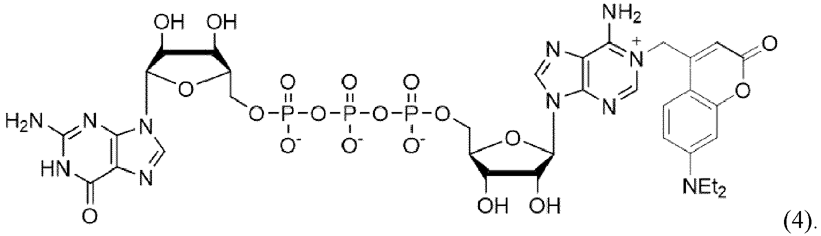

8. The photocleavable nucleoside or nucleotide analog according to any one of claims 2 to 4, wherein the photocleavable nucleotide analog has a structure according to formula (1) or formula (3) as given as follows or salts thereof: 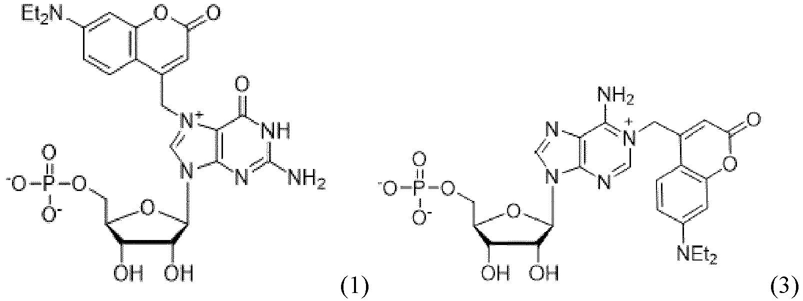 wherein Et is ethyl.

9. The photocleavable 5'-cap analog according to any one of claims 1 or 3-7 for use as a medicament or for use in the diagnosis and/or treatment of disorders in connection with dysregulation in mRNA translation, particularly for use in the treatment of pain, cancer, autoimmune diseases or virus infection.

10. The photocleavable nucleoside or nucleotide analog according to any one of claims 2-4 or 8 for use in metabolic labelling.

11. An RNA molecule comprising a photocleavable 5'-cap analog according to any one of claims 1 or 3-7.

12. The RNA molecule according to claim 11 for use as a medicament or for use in the diagnosis and/or treatment of disorders in connection with dysregulation in mRNA translation, particularly for use as a vaccine or for use in protein replacement therapy or for use in the treatment of pain, cancer, autoimmune diseases or virus infection.

13. A diagnostic or pharmaceutical composition comprising a photocleavable 5'-cap analog according to any one of claims 1 or 3-7 or an RNA molecule according claim 9 and a pharmaceutically acceptable carrier.

14. A diagnostic composition comprising a photocleavable nucleoside or nucleotide analog according to any one of claims 2-4 or 8 and a pharmaceutically acceptable carrier.

15. Use of a photocleavable 5'-cap analog according to any one of claims 1 or 3-7 in a method of synthesising an RNA molecule, or for inhibiting cap-binding proteins or cap-degrading enzymes.
